# EUROPEAN PATENT SPECIFICATION

(11) **EP 0 643 767 B1**
(45) Date of publication and mention of the grant of the patent: **22.07.1998**
(21) Application number: 91919612.1
(22) Date of filing: 18.10.1991
(51) Int. Cl.: C12N 15/00, C07K 14/00, A61K 38/00

(54) **OSTEOGENIC PEPTIDES**
OSTEOGENE PEPTIDE
PEPTIDES OSTEOGENIQUES

(30) Priority: 18.10.1990 US 599543
(43) Date of publication of application: 22.03.1995
(73) Proprietor: STRYKER CORPORATION, Kalamazoo, Michigan 49003-4085 (US)
(72) Inventor: OPPERMANN, Hermann, Medway, MA 02053 (US); OZKAYNAK, Engin, Milford, MA 01757 (US); REUGER, David, C., Hopkinton, Massachusetts 01748 (US); KUBERASAMPATH, Thangavel, Medway, MA 02053 (US)
(74) Representative: Hutchins, Michael Richard
(86) International application number: US9107635
(87) International publication number: WO9207073

(56) References cited:
- WO-A-89/09788
- WO-A-90/11366
- EMBO Journal, volume 9, no. 7, 1990, Oxford University Press (Eynsham, Oxford, GB E. Ozkaynak et al.: "OP-1 cDNA encodes an osteogenic protein in the TGF- beta familiy", pages 2085-2093, see the whole article

## Description

### Background of the Invention

This invention relates to the subject matter of the claims.

Mammalian bone tissue is known to contain one or more proteinaceous materials, presumably active during growth and natural bone healing, which can induce a developmental cascade of cellular events resulting in endochondral bone formation. This active factor (or factors) has variously been referred to in the literature as bone morphogenetic or morphogenic protein, bone inductive protein, osteogenic protein, osteogenin, or osteoinductive protein.

The developmental cascade of bone differentiation consists of recruitment of mesenchymal cells, proliferation of progenitor cells, calcification of cartilage, vascular invasion, bone formation, remodeling, and finally marrow differentiation (Reddi (1981) Collagen Rel. Res. 1:209-226).

Though the precise mechanisms underlying these phenotypic transformations are unclear, it has been shown that the natural endochondral bone differentiation activity of bone matrix can be dissociatively extracted and reconstituted with inactive residual collagenous matrix to restore full bone induction activity (Sampath and Reddi, (1981) Proc. Natl. Acad. Sci. USA 78:7599-7603). This provides an experimental method for assaying protein extracts for their ability to induce endochondral bone in vivo. Several species of mammals produce closely related protein as demonstrated by cross species implant experiments (Sampath and Reddi (1983) Proc. Natl. Acad. Sci. USA 80:6591-6595).

The potential utility of these proteins has been recognized widely. It is contemplated that the availability of the protein would revolutionize orthopedic medicine, certain types of plastic surgery, and various periodontal and craniofacial reconstructive procedures.

The observed properties of these protein fractions have induced an intense research effort in various laboratories directed to isolating and identifying the pure factor or factors responsible for osteogenic activity. The current state of the art of purification of osteogenic protein from mammalian bone is disclosed by Sampath et al. ((1987) Proc. Natl. Acad. Sci. USA 84: 7109-7113). Urist et al. (1984) Proc. Soc. Exp. Biol. Med. 173: 194-199 disclose a human osteogenic protein fraction which was extracted from demineralized cortical bone by means of a calcium chloride-urea inorganic-organic solvent mixture, and retrieved by differential precipitation in guanidine-hydrochloride and preparative gel electrophoresis. The authors report that the protein fraction has an amino acid composition of an acidic polypeptide and a molecular weight in a range of 17-18 kD.

Urist et al. (1984) Proc. Natl. Acad. Sci. USA 81: 371-375 disclose a bovine bone morphogenetic protein extract having the properties of an acidic polypeptide and a molecular weight of approximately 18 kD. The authors reported that the protein was present in a fraction separated by hydroxyapatite chromatography, and that it induced bone formation in mouse hindquarter muscle and bone regeneration in trephine defects in rat and dog skulls. Their method of obtaining the extract from bone results in ill-defined and impure preparations.

European Patent Application Serial No. 148,155, published October 7, 1985, purports to disclose osteogenic proteins derived from bovine, porcine, and human origin. One of the proteins, designated by the inventors as a P3 protein having a molecular weight of 22-24 kD, is said to have been purified to an essentially homogeneous state. This material is reported to induce bone formation when implanted into animals.

International Application No. PCT/087/01537, published January 14, 1988, discloses an impure fraction from bovine bone which has bone induction qualities. The named applicants also disclose putative "bone inductive factors" produced by recombinant DNA techniques. Four DNA sequences were retrieved from human or bovine genomic or cDNA libraries and expressed in recombinant host cells. While the applicants stated that the expressed proteins may be bone morphogenic proteins, bone induction was not demonstrated, suggesting that the recombinant proteins are not osteogenic. The same group reported subsequently (Science, 242:1528, Dec, 1988) that three of the four factors induce cartilage formation, and postulate that bone formation activity "is due to a mixture of regulatory molecules" and that "bone formation is most likely controlled ... by the interaction of these molecules." Again, no bone induction was attributed to the products of expression of the cDNAs. See also Urist et al., EP0,212,474 entitled Bone Morphogenic Agents.

Wang et al. (1988) Proc. Nat. Acad. Sci. USA 85: 9484-9488 discloses the purification of a bovine bone morphogenetic protein from guanidine extracts of demineralized bone having cartilage and bone formation activity as a basic protein corresponding to a molecular weight of 30 kD determined from gel elution. Purification of the protein yielded proteins of 30, 18 and 16 kD which, upon separation, were inactive. In view of this result, the authors acknowledged that the exact identity of the active material had not been determined.

Wang et al. (1990) Proc. Nat. Acad. Sci. USA 87: 2220-2227 describes the expression and partial purification of one of the cDNA sequences described in PCT 87/01537. Consistent cartilage and/or bone formation with their protein requires a minimum of 600 ng of 50% pure material.

International Application No. PCT/89/04458 published April 19, 1990 (Int. Pub. No. W090/003733), describes the purification and analysis of a family of osteogenic factors called "P3 OF 31-34". The protein family contains at least four proteins, which are characterized by peptide fragment sequences. The impure mixture P3 OF 31-34 is assayed for osteogenic activity. The activity of the individual proteins is neither assessed nor discussed.

Described herein are polypeptide chains useful as subunits of dimeric osteogenic proteins capable of endochondral bone formation in allogenic and xenogenic implants in mammals, including humans. Also described are genes encoding these polypeptide chains and methods for the production of osteogenic proteins comprising these polypeptide chains using recombinant DNA techniques, as well as antibodies capable of binding specifically to these proteins.

These and other objects and features of the invention will be apparent from the description, drawings, and claims which follow.

### Summary of the Invention

This invention concerns the subject matter of the claims, and provides novel polypeptide chains useful as either one or both subunits of dimeric osteogenic proteins which, when implanted in a mammalian body in association with a matrix, can induce at the locus of the implant the full developmental cascade of endochondral bone formation and bone marrow differentiation.

A key to these developments was the elucidation of amino acid sequence and structure data of native bovine osteogenic protein. A protocol was developed which results in retrieval of active, substantially pure osteogenic protein from bovine bone having a half-maximum bone forming activity of about 0.8 to 1.0 ng per mg of implant. The availability of the material enabled the inventors to elucidate key structural details of the protein necessary to achieve bone formation. Knowledge of the protein's amino acid sequence and other structural features enabled the identification and cloning of native genes in the human genome.

Consensus DNA sequences based on partial sequence data and observed homologies with regulatory proteins disclosed in the literature were used as probes for extracting genes encoding osteogenic protein from human genomic and cDNA libraries. One of the consensus sequences was used to isolate a previously unidentified gene which, when expressed, encoded a protein comprising a region capable of inducing endochondral bone formation when properly modified, incorporated in a suitable matrix, and implanted as disclosed herein. The gene, called "hOP1" or "OP-1" (human OP-1), is described in greater detail in copending U.S. 422,699, the disclosure of which is herein incorporated by reference.

In its native form, hOP1 expression yields an immature translation product ("hOP1-PP", where "PP" refers to "prepro form") of about 400 amino acids that subsequently is processed to yield a mature sequence of 139 amino acids ("OP1-18"). The active region (functional domain) of the protein comprises the C-terminal 97 amino acids of the hOP1 sequence ("OPS"). A long active sequence is OP7 (comprising the C-terminal 102 amino acids).

Further probing of mammalian cDNA libraries (human and mouse) with sequences specific to hOP1 also has identified novel OP1-like sequences herein referred to as "OP2" ("hOP2" or "mOP2"). The OP2 proteins share significant amino acid sequence homology, approximately 74%, with the active region of the OP1 proteins (e.g., OP7), and less homology with the intact mature form (e.g., OP1-18, 58% amino acid homology).

The amino acid sequence of the osteogenic proteins disclosed herein also share significant homology with various of the regulatory proteins on which the consensus probe was modeled. In particular, the proteins share significant homology in their C-terminal sequences, which comprise the active region of the osteogenic proteins. (Compare, for example, OP7 with DPP from Drosophila and Vgl from Xenopus. See, for example, U.S. Pat. No. 5,011,691). In addition, these proteins share a conserved six or seven cysteine skeleton in this region (e.g., the linear arrangement of these C-terminal cysteine residues is conserved in the different proteins.) See, for example, OP7, whose sequence defines the seven cysteine skeleton, or OPS, whose sequence defines the six cysteine skeleton. The OP2 proteins also contain an additional cysteine residue within this region.

Thus, in one preferred aspect, the invention comprises osteogenic proteins comprising a polypeptide chain comprising an amino acid sequence described by Seq. ID No. 3 or 5 such that a dimeric protein comprising this polypeptide chain has a conformation capable of inducing endochondral bone formation when implanted in a mammal in association with a suitable matrix. Useful proteins include the fuLl-length protein and proteins comprising the functional domain described by the C-terminal.

In addition, the invention is not limited to these specific constructs. Thus, the osteogenic proteins of this invention comprising any of these polypeptide chains may include forms having varying glycosylation patterns, varying N-termini, a family of related proteins having regions of amino acid sequence homology which may be naturally occurring or biosynthetically derived, and active truncated forms of the native amino acid sequence, produced by expression of recombinant DNA in procaryotic or eucaryotic host cells. Active squances useful as osteogenic proteins of this invention are envisioned to include proteins capable of inducing endochondral bone formation when implanted in a mammal in association wiht a matrix and having at least a 70% sequence homology, preferably at least 80%, with the amino acid sequence of OPS.

The novel polypeptide chains and the osteogenic proteins they comprise can be expressed from intact or truncated cDNA or from synthetic DNAs in procaryotic or eucaryotic host cells, and then purified, cleaved, refolded, dimerized, and implanted in experimental animals. Currently preferred host cells include E.coli or mammalian cells, such as CHO, COS or 35C cells. The osteogenic protein of the invention may include forms having varying glycosylation patterns, varying N-termini, a family of related proteins having regions of amino acid sequence homology, and active truncated forms of native or biosynthetic proteins, produced by expression of recombinant DNA in host cells.

Thus, in view of this disclosure, skilled genetic engineers can isolate genes from cDNA or genomic libraries of various different species which encode appropriate amino acid sequences, or construct DNAs from oligonucleotides, and then can express them in various types of host cells, including both procaryotes and eucaryotes, to produce large quantities of active proteins capable of inducing bone formation in mammals including humans. In view of this disclosure, those skilled in the art, using standard immunology techniques also may create antibodies capable of binding specifically to the osteogenic proteins disclosed herein, including fragments thereof.

The osteogenic proteins are useful in clinical applications in conjunction with a suitable delivery or support system (matrix). The matrix is made up of particles of porous materials. The pores must be of a dimension to permit progenitor cell migration and subsequent differentiation and proliferation. The particle size should be within the range of 70 - 850 mm, preferably 150mm - 420mm. It may be fabricated by close packing particulate material into a shape spanning the bone defect, or by otherwise structuring as desired a material that is biocompatible (non-inflammatory) and, biodegradable in vivo to serve as a "temporary scaffold" and substratum for recruitment of migratory progenitor cells, and as a base for their subsequent anchoring and proliferation. Currently preferred carriers include particulate, demineralized, guanidine extracted, species-specific (allogenic) bone, and specially treated particulate, protein extracted, demineralized, xenogenic bone. Optionally, such xenogenic bone powder matrices also may be treated with proteases such as trypsin and/or fibril modifying agents to increase the intraparticle intrusion volume and surface area. Useful agents include solvents such as dichloromethane, trichloroacetic acid, acetonitrile and acids such as trifluoroacetic acid and hydrogen fluoride. Alternatively, the matrix may be treated with a hot aqueous medium having a temperature within the range of about 37°C to 75°C, including a heated acidic aqueous medium. Other potentially useful matrix materials comprise collagen, homopolymers and copolymers of glycolic acid and lactic acid, hydroxyapatite, tricalcium phosphate and other calcium phosphates.

The osteogenic proteins and implantable osteogenic devices enabled and disclosed herein will permit the physician to obtain optimal predictable bone formation to correct, for example, acquired and congenital craniofacial and other skeletal or dental anomalies (Glowacki et al. (1981) Lancet 1:959-963). The devices may be used to induce local endochondral bone formation in non-union fractures as demonstrated in animal tests, and in other clinical applications including dental and periodontal applications where bone formation is required. Another potential clinical application is in cartilage repair, for example, in the treatment of osteoarthritis.

### Brief Description of the Drawing

The foregoing and other objects of this invention, the various features thereof, as well as the invention itself, may be more fully understood from the following description, when read together with the accompanying drawings, in which:
FIGURE 1 compares the amino acid sequences of the mature mOP-2 and hOP-2 polypeptide chains: hOP2-A and mOP2-A; and
FIGURE 2 compares the amino acid sequences of the mature OP1 and OP2 polypeptide chains: OP1-18, mOP1-S, hOP2-A and mOP2-A.

### Description

Purification protocols first were developed which enabled isolation of the osteogenic protein present in crude protein extracts from mammalian bone. (See PCT WO 89/09787, published 19-OCT-89, and U.S. Serial No. 179,406 filed April 8, 1988, now U.S. Patent No. 4,968,950). The development of the procedure, coupled with the availability of fresh calf bone, enabled isolation of substantially pure bovine osteogenic protein (bOP). bOP was characterized significantly; its ability to induce cartilage and ultimately endochondral bone growth in cat, rabbit, and rat were demonstrated and studied; it was shown to be able to induce the full developmental cascade of bone formation previously ascribed to unknown protein or proteins in heterogeneous bone extracts. This dose dependent and highly specific activity was present whether or not the protein was glycosylated (see Sampath et al., (1990) J. Biol. Chem. 265: 13198-13205). Sequence data obtained from the bovine materials suggested probe designs which were used to isolate human genes. The OP human counterpart proteins have now been expressed and extensively characterized.

These discoveries enabled preparation of DNAs encoding totally novel, non-native protein constructs which individually as homodimers and combined with other species as heterodimers are capable of producing true endochondral bone (see PCT WO 09788, published 19-OCT-89, and US Serial No. 315,342, filed 23-FEB-89, now U.S. Patent No. 5,011,691). They also permitted expression of the natural material, truncated forms, muteins, analogs, fusion proteins, and various other variants and constructs, from cDNAs and genomic DNAs retrieved from natural sources or from synthetic DNA produced using the techniques disclosed herein and using automated, commercially available equipment. The DNAs may be expressed using well established molecular biology and recombinant DNA techniques in procaryotic or eucaryotic host cells, and may be oxidized and refolded in vitro if necessary, to produce biologically active protein.

One of the DNA sequences isolated from human genomic and cDNA libraries encoded a previously unidentified gene, referred to herein as OP1. The protein encoded by the isolated DNA was identified originally by amino acid homology with proteins in the TGF-β family. Consensus splice signals were found where amino acid homologies ended, designating exon-intron boundaries. Three exons were combined to obtain a functional TGF-β-like domain containing seven cysteines. (See, for example, U.S. Patent No. 5,011,691, or Ozkaynak, E. et al., (1990) EMBO. 9: 2085-2093).

The full-length cDNA sequence for hOP1, and its encoded "prepro" form "hOP1-PP," which includes an N-terminal signal peptide sequence, are disclosed in Seq. ID No. 1 (residues 1-431). The mature form of the hOP1 protein expressed in mammalian cells, "OP1-18", is described by amino acid residues 293-431 of Seq. ID No. 1. The full length form of hOP1, as well as various truncated forms of the gene, and fused genes, have been expressed in E. coli and numerous mammalian cells (see, for example, published PCT application WO 91/05802, published 2-MAY-91) and all have been shown to have osteogenic activity when implanted in a mammal in association with a suitable matrix.

Given the foregoing amino acid and DNA sequence information, various nucleic acids (RNAs and DNAs) can be constructed which encode at least the active region of the hOP1 protein (e.g., OPS or OP7) and various analogs thereof (including allelic and species variants and those containing genetically engineered mutations), as well as fusion proteins, truncated forms of the mature proteins, and similar constructs. Moreover, DNA hybridization probes can be constructed from fragments of the hOP1 DNA or designed de novo based on the hOP1 DNA or amino acid sequence. These probes then can be used to screen different genomic and cDNA libraries to identify additional osteogenic proteins.

The DNAs can be produced by those skilled in the art using well known DNA manipulation techniques involving genomic and cDNA isolation, construction of synthetic DNA from synthesized oligonucleotides, and cassette mutagenesis techniques. 15-100mer oligonucleotides may be synthesized on a Biosearch DNA Model 8600 Synthesizer, and purified by polyacrylamide gel electrophoresis (PAGE) in Tris-Borate-EDTA buffer. The DNA then may be electroeluted from the gel. Overlapping oligomers may be phosphorylated by T4 polynucleotide kinase and ligated into larger blocks which may also be purified by PAGE.

DNAs used as hybridization probes may be labelled (e.g., as with a radioisotope, by nick-translation) and used to identify clones in a given library containing DNA to which the probe hybridizes, following techniques well known in the art. The libraries may be obtained commercially or they may constructed de novo using conventional molecular biology techniques. Further information on DNA library construction and hybridization techniques can be found in numerous texts known to those skilled in the art. See, for example, F.M. Ausubel., ed., Current Protocols in Molecular Biology-Vol. 1, (1989). In particular, see unit 5, "Construction of Recombinant DNA Libraries" and Unit 6, "Screening of Recombinant Libraries."

The DNA from appropriately identified clones then can be isolated, subcloned (preferably into an expression vector), and sequenced. Plasmids containing sequences of interest then can be transfected into an appropriate host cell for protein expression and further characterization. The host may be a procaryotic or eucaryotic cell since the former's inability to glycosylate protein will not destroy the protein's osteogenic activity. Useful host cells include E. coli, Saccharomyces, the insect/baculovirus cell system, myeloma cells, and various mammalian cells. The vector additionally may encode various sequences to promote correct expression of the recombinant protein, including transcription promoter and termination sequences, enhancer sequences, preferred ribosome binding site sequences, preferred mRNA leader sequences, preferred signal sequences for protein secretion, and the like. The DNA sequence encoding the gene of interest also may be manipulated to remove potentially inhibiting sequences or to minimize unwanted secondary structure formation. The recombinant osteogenic protein also may be expressed as a fusion protein. After being translated, the protein may be purified from the cells themselves or recovered from the culture medium. All biologically active protein forms comprise dimeric species joined by disulfide bonds or otherwise associated, produced by oxidizing and refolding one or more of the various recombinant polypeptide chains within an appropriate eucaryotic cell or in vitro after expression of individual subunits. A detailed description of osteogenic protein expressed from recombinant DNA in E. coli is disclosed in U.S. Serial No. 660,162, filed 27-FEB-91, the disclosure of which incorporated by reference herein. A detailed description of osteogenic protein expressed from recombinant DNA in numerous different mammalian cells is disclosed in PCT WO91/05802, also incorporated herein by reference.

Finally, in view of the disclosure made herein, and using standard methodologies known in the art, persons skilled in the art can raise polyclonal and monoclonal antibodies against all or part of a polypeptide chain disclosed herein, such that the antibodies are capable of binding specifically to an epitope on the polypeptide chain. Useful protocols can be found in, for example, Molecular Cloning-A Laboratory Manual (Sambrook et al. eds., Cold Spring Harbor Press 2nd ed., 1989). See Book 3, Section 18.

### Exemplification

In an effort to identify additional DNA sequences encoding osteogenic proteins, a hybridization probe specific to the C-terminus of the DNA of mature OP-1 was prepared using a StuI-EcoR1 digest fragment of OP-1 (base pairs 1034-1354 in Sequence ID No. 1), and labelled with ³²P by nick translation, as described in the art. As disclosed supra, the OP1 C-terminus encodes a key functional domain, e.g., the "active region" for osteogenic activity. The C-terminus also is the region of the protein whose amino acid sequence shares specific amino acid sequence homology with particular proteins in the TGF-β super-family of regulatory proteins, and which includes the conserved cysteine skeleton.

Approximately 7 x 10⁵ phages of an oligo(dT) primed 17.5 days p.c. mouse embryo 5' stretch cDNA (gt10) library (Clonetech, Inc., Palo Alto, CA) was screened with the labelled probe. The screen was performed using the following stringent hybridization conditions: 40% formamide, 5 x SSPE, 5 x Denhart's solution, 0.1% SDS, at 37°C overnight, and washing in 0.1 x SSPE, 0.1% SDS at 50°C.

Five recombinant phages were purified over three rounds of screening. Phage DNA was prepared from all five phages, subjected to an EcoR1 digest, subcloned into the EcoR1 site of a common pUC-type plasmid modified to allow single strand sequencing, and sequenced using means well known in the art.

Two different DNAs were identified by this procedure. One DNA, referred to herein as mOP1, has substantial homology to the mature form of OP1 (about 98%). A second DNA, encoding the C-terminus of a related gene and referred to herein as mOP2, also was identified by this procedure. The N-terminus of the gene encoding mOP2 was identified subsequently by screening a second mouse cDNA library (Mouse PCC4 cDNA (ZAP) library, Stratagene, Inc., La Jolla, CA).

Mouse OP2 (mOP2) protein shares significant amino acid sequence homology with the amino acid sequence of the hOP1 active region, e.g., OPS or OP7, about 74% homology, and less homology with the intact mature form, e.g., OP1-18, about 58% homology. The cDNA sequence, and the encoded amino acid sequence, for the full length mOP-2 protein is depicted in Sequence ID No. 3. The full-length form of the protein is referred to as the prepro form of mOP-2 ("mOP2-PP"), and includes a signal peptide sequence at its N-terminus. The amino acid sequence Leu-Ala-Leu-Cys-Ala-Leu (amino acid residues 13-18 of Sequence ID No. 3) is believed to constitute the cleavage site for the removal of the signal peptide sequence, leaving an intermediate form of the protein, the "pro" form, to be secreted from the expressing cell. The amino acid sequence Arg-Ala-Pro-Arg-Ala (amino acid residues 255-259 of Sequence ID No. 3) is believed to constitute the cleavage site that produces the mature form of the protein, herein referred to as "mOP2-A", and described by residues 259-397 of Seq. ID No. 3. Residues 301-397 of Seq. ID No. 3 correspond to the region defining the conserved six cysteine skeleton. Residues 296-397 of Seq. ID No. 3 correspond to the region defining the conserved seven cysteine skeleton.

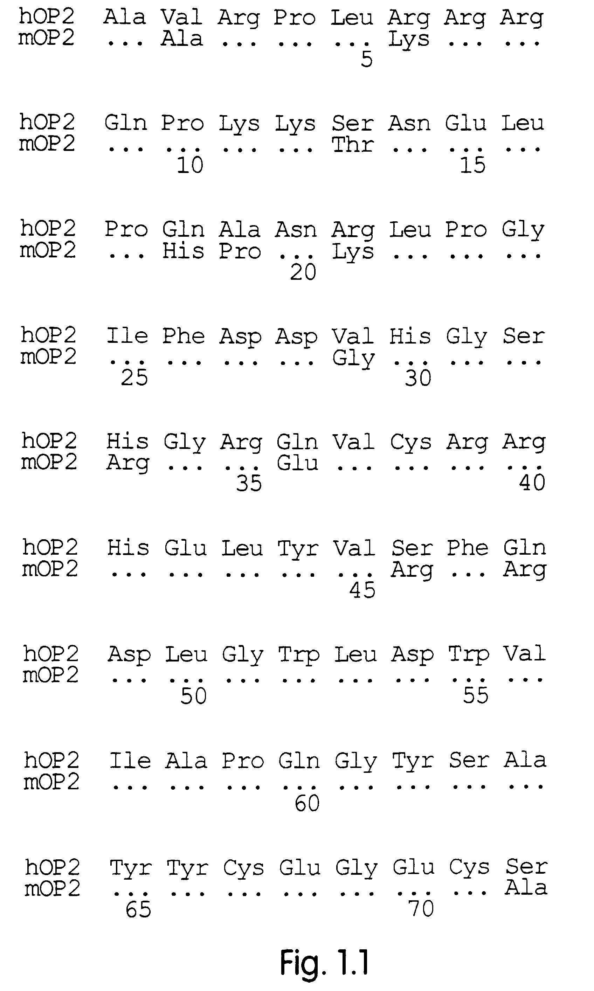

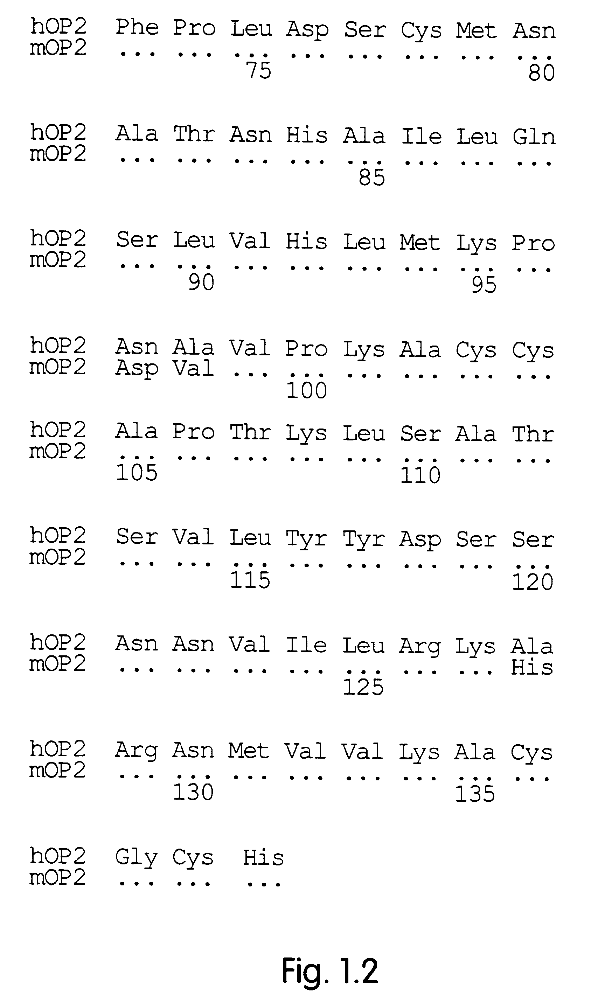

Using a probe prepared from the pro region of mOP2 (an EcoR1-BamH1 digest fragment, bp 467-771 of Sequence ID No. 3), a human hippocampus library was screened (human hippocampus cDNA lambda (ZAP II library Stratagene, Inc., La Jolla, CA) following essentially the same procedure as for the mouse library screens. The procedure identified the N-terminus of a novel DNA encoding an amino acid sequence having substantial homology with mOP2. The C-terminus of the gene subsequently was identified by probing a human genomic library (in lambda phage EMBL-3, Clonetech, Inc., Palo Alto, CA) with a labelled fragment from the novel human DNA in hand. The novel polypeptide chain encoded by this DNA is referred to herein as hOP2 protein, and shares almost complete amino acid identity (about 92% amino acid sequence homology) with mOP2-A (see Fig. 1 and infra).

The cDNA sequence, and the encoded amino acid sequence, for the prepro form of hOP2, "hOP2-PP", is described in Sequence ID No. 5. This full-length form of the protein also includes a signal peptide sequence at its N-terminus. The amino acid sequence Leu-Ala-Leu-Cys-Ala-Leu (amino acid residues 13-18 of Sequence ID No. 5) is believed to constitute the cleavage site for the removal of the signal peptide sequence, leaving an intermediate form of the protein, the "pro" form, to be secreted from the expressing cell. The amino acid sequence Arg-Thr-Pro-Arg-Ala (amino acid residues 257-261 of Sequence ID No. 5) is believed to constitute the cleavage site that produces what is believed to be the mature form of the protein, herein referred to as hOP2-A" and described by residues 261-399 of Seq. ID No. 5.

Additional mature species of hOP2 thought to be active include truncated sequences, "hOP2-P" (described by residues 264-399 of Seq. ID No. 5) and "hOP2-R" (described by residues 267-399 of Seq. ID No. 5), and a slightly longer sequence ("hOP2-S", described by residues 240-399 of Seq. ID No. 5). Residues 303-399 of Seq. ID No. 5 correspond to the region defining the conserved six cysteine skeleton. Residues 298-399 of Seq. ID No. 5 correspond to the region defining the conserved seven cystein skeleton.

It should be noted that the nucleic acid sequence encoding the N-terminus of the prepro form of both mOP2 and hOP2 is rich in guanidine and cytosine base pairs. As will be appreciated by those skilled in the art, sequencing such a "G-C rich" region can be problematic, due to stutter and/or band compression. Accordingly, the possibility of sequencing errors in this region can not be ruled out. However, the definitive amino acid sequence for these and other, similarly identified proteins can be determined readily by expressing the protein from recombinant DNA using, for example, any of the means disclosed herein, and sequencing the polypeptide chain by conventional peptide sequencing methods well known in the art.

Figure 1 compares the amino acid sequences of mature mOP2 and hOP2. Identity is indicated by three dots (...) in the mOP2 sequence. As is evident from the figure, the amino acid sequence homology between the mature forms of these two proteins is substantial (92% homology between the mature sequences, about 95% homology within the C-terminal active region (e.g., residues 38-139 or 42-139 of Fig. 1.)

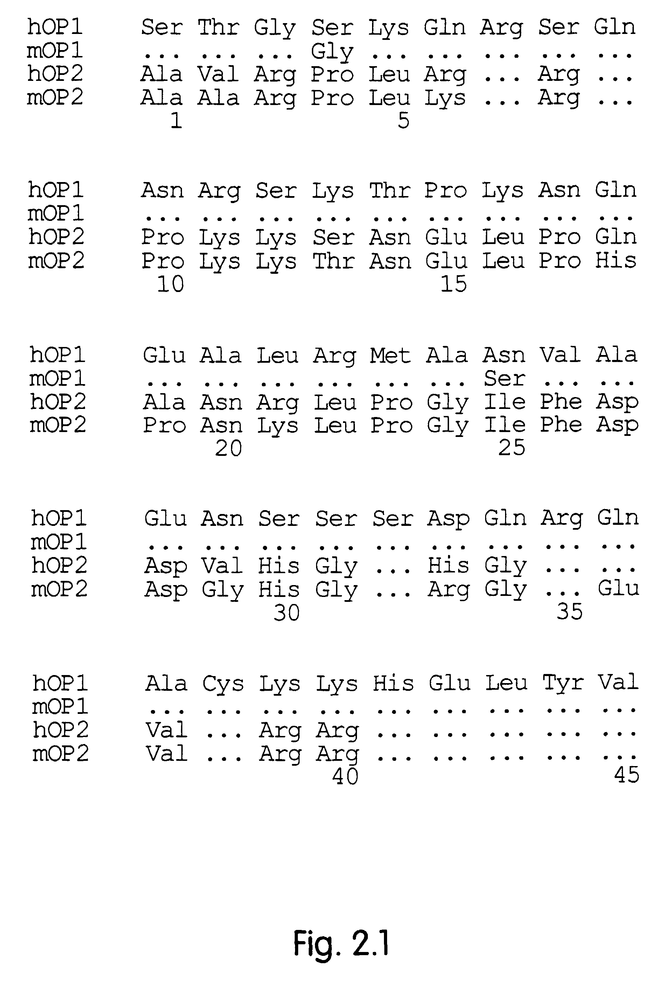

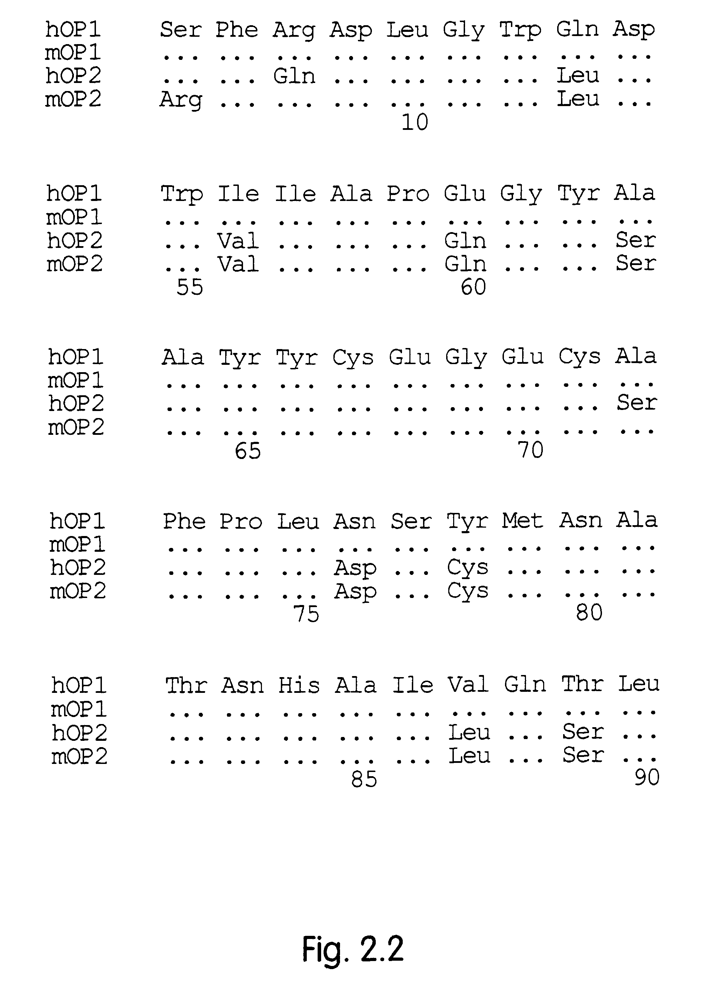

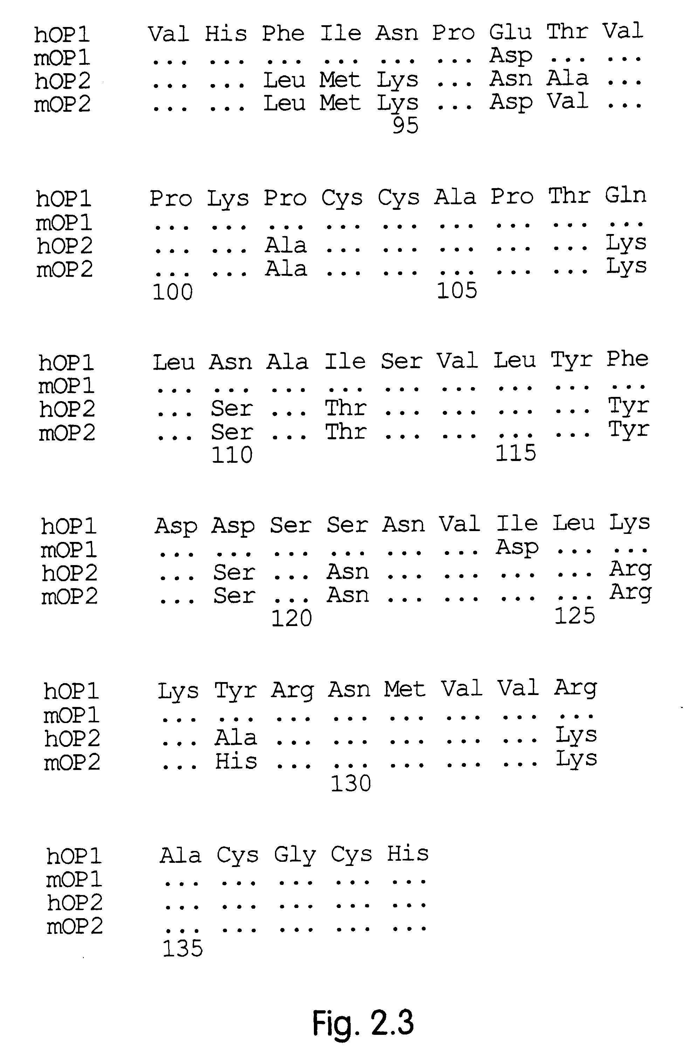

Fig. 2 compares the amino acid sequences for the mature forms of all four species of OP1 and OP2 proteins. Here again, identity is indicated by three dots (...). Like the mOP2 protein, the hOP2 protein shares significant homology (about 74%) with the amino acid sequence defining the OP1 active region (OPS or OP7, residues 43-139 and 38-139, respectively, in Fig. 2), and less homology with OP1-18 (about 58% homology). Both OP2 proteins share the conserved seven cysteine skeleton seen in the OP1 proteins. In addition, the OP2 proteins comprise an eighth cysteine residue within this region (see position 78 in FIG. 2).

A preferred generic amino acid sequence useful as a subunit of a dimeric osteogenic protein capable of inducing endochondral bone or cartilage formation when implanted in a mammal in association with a matrix, and which incorporates the maximum homology between the identified OP1 and OP2 proteins, can be described by the sequence referred to herein as "OPX", described below and in Seq. No.7. and wherein Xaa at res. 2 = (Lys or Arg); Xaa at res. 3 = (Lys or Arg); Xaa at res. 9 = (Ser or Arg); Xaa at res. 11 = (Arg or Gln); Xaa at res. 16 = (Gln or Leu); Xaa at res. 19 = (Ile or Val); Xaa at res. 23 = (Glu or Gln); Xaa at res. 26 = (Ala or Ser); Xaa at res. 35 = (Ala or Ser); Xaa at res. 39 = (Asn or Asp); Xaa at res. 41 = (Tyr or Cys); Xaa at res. 50 = (Val or Leu); Xaa at res. 52 = (Ser or Thr); Xaa at res. 56 = (Phe or Leu); Xaa at res. 57 = (Ile or Met); Xaa at res. 58 = (Asn or Lys); Xaa at res. 60 = (Glu, Asp or Asn); Xaa at res. 61 = (Thr, Ala or Val); Xaa at res. 65 = (Pro or Ala); Xaa at res. 71 = (Gln or Lys); Xaa at res. 73 = (Asn or Ser); Xaa at res. 75 = (Ile or Thr); Xaa at res. 80 = (Phe or Tyr); Xaa at res. 82 = (Asp or Ser); Xaa at res. 84 = (Ser or Asn); Xaa at res. 87 = (Ile or Asp); Xaa at res. 89 = (Lys or Arg); Xaa at res. 91 = (Tyr, Ala or His); and Xaa at res. 97 = (Arg or Lys).

The high degree of homology exhibited between the various OP1 and OP2 proteins suggests that the novel osteogenic proteins identified herein will purify essentially as OP1 does, or with only minor modifications of the protocols disclosed for OP1. Similarly, the purified mOP1, mOP2, and hOP2 proteins are predicted to have an apparent molecular weight of about 18 kDa as reduced single subunits, and an apparent molecular weight of about 36 kDa as oxidized dimers, as determined by comparison with molecular weight standards on an SDS-polyacrylamide electrophoresis gel. Unglycosylated dimers (e.g., proteins produced by recombinant expression in E. coli) are predicted to have an apparent molecular weight of about 27 kDa. There appears to be one potential N glycosylation site in the mature forms of the mOP2 and hOP2 proteins.

The identification of osteogenic proteins having an active region comprising eight cysteine residues also allows one to construct osteogenic polypeptide chains patterned after either of the following template amino acid sequences, or to identify additional osteogenic proteins having this sequence. The template sequences contemplated are "OPX-7C", comprising the conserved six cysteine skeleton plus the additional cysteine residue identified in the OP2 proteins, and "OPX-8C", comprising the conserved seven cysteine skeleton plus the additional cysteine residue identified in the OP2 proteins. The OPX-7C and OPX-8C sequences are described below and in Seq. ID Nos. 8 and 9, respectively. Each Xaa in these template sequences independently represents one of the 20 naturally-occurring L-isomer, α-amino acids, or a derivative thereof. Biosynthetic constructs patterned after this template readily are constructed using conventional DNA synthesis or peptide synthesis techniques well known in the art. Once constructed, osteogenic proteins comprising these polypeptide chains can be tested as disclosed herein. "OPX-8C" (Sequence ID No. 9 comprising additional five residues at the N-terminus, including a conserved cysteine residue):

### MATRIX PREPARATION

### A. General Consideration of Matrix Properties

The currently preferred carrier material is a xenogenic bone-derived particulate matrix treated as disclosed herein. This carrier may be replaced by either a biodegradable-synthetic or synthetic-inorganic matrix (e.g., hydroxylapatite (HAP), collagen, tricalcium phosphate or polylactic acid, polyglycolic acid and various copolymers thereof.)

Studies have shown that surface charge, particle size, the presence of mineral, and the methodology for combining matrix and osteogenic protein all play a role in achieving successful bone induction. Perturbation of the charge by chemical modification abolishes the inductive response. Particle size influences the quantitative response of new bone; particles between 75 µm and 420 µm elicit the maximum response. Contamination of the matrix with bone mineral will inhibit bone formation. Most importantly, the procedures used to formulate OP onto the matrix are extremely sensitive to the physical and chemical state of both the osteogenic protein and the matrix.

The sequential cellular reactions in the interface of the bone matrix/osteogenic protein implants are complex. The multistep cascade includes: binding of fibrin and fibronectin to implated matrix, chemotaxis of cells, proliferation of fibroblasts, differentiation into chondroblasts, cartilage formation, vascular invasion, bone formation, remodeling, and bone marrow differentiation.

A successful carrier for osteogenic protein must perform several important functions. It must bind osteogenic protein and act as a slow release delivery system, accommodate each step of the cellular response during bone development, and protect the osteogenic protein from nonspecific proteolysis. In addition, selected materials must be biocompatible in vivo and preferably biodegradable; the carrier must act as a temporary scaffold until replaced completely by new bone. Polylactic acid (PLA), polyglycolic acid (PGA), and various combinations have different dissolution rates in vivo. In bones, the dissolution rates can vary according to whether the implant is placed in cortical or trabecular bone.

Matrix geometry, particle size, the presence of surface charge, and the degree of both intra-and-inter-particle porosity are all important to successful matrix performance. It is preferred to shape the matrix to the desired form of the new bone and to have dimensions which span non-union defects. Rat studies show that the new bone is formed essentially having the dimensions of the device implanted.

The matrix may comprise a shape-retaining solid made of loosely adhered particulate material, e.g., with collagen. It may also comprise a molded, porous solid, or simply an aggregation of close-packed particles held in place by surrounding tissue. Masticated muscle or other tissue may also be used. Large allogenic bone implants can act as a carrier for the matrix if their marrow cavities are cleaned and packed with particle and the dispersed osteogenic protein.

The preferred matrix material, prepared from xenogenic bone and treated as disclosed herein, produces an implantable material useful in a variety of clinical settings. In addition to its use as a matrix for bone formation in various orthopedic, periodontal, and reconstructive procedures, the matrix also may be used as a sustained release carrier, or as a collagenous coating for implants. The matrix may be shaped as desired in anticipation of surgery or shaped by the physician or technician during surgery. Thus, the material may be used for topical, subcutaneous, intraperitoneal, or intramuscular implants; it may be shaped to span a nonunion fracture or to fill a bone defect. In bone formation or conduction procedures, the material is slowly absorbed by the body and is replaced by bone in the shape of or very nearly the shape of the implant.

Various growth factors, hormones, enzymes, therapeutic compositions, antibiotics, and other body treating agents also may be absorbed onto the carrier material and will be released over time when implanted as the matrix material is slowly absorbed. Thus, various known growth factors such as EGF, PDGF, IGF, FGF, TGF-α, and TGF-β may be released in vivo. The material can be used to release chemotherapeutic agents, insulin, enzymes, or enzyme inhibitors.

### B. Bone-Derived Matrices

### 1. Preparation of Demineralized Bone

Demineralized bone matrix, preferably bovine bone matrix, is prepared by previously published procedures (Sampath and Reddi (1983) Proc. Natl. Acad. Sci. USA 80:6591-6595). Bovine diaphyseal bones (age 1-10 days) are obtained from a local slaughterhouse and used fresh. The bones are stripped of muscle and fat, cleaned of periosteum, demarrowed by pressure with cold water, dipped in cold absolute ethanol, and stored at -20°C. They are then dried and fragmented by crushing and pulverized in a large mill. Care is taken to prevent heating by using liquid nitrogen. The pulverized bone is milled to a particle size in the range of 70-850 µm, preferably 150-420 µm, and is defatted by two washes of approximately two hours duration with three volumes of chloroform and methanol (3:1). The particulate bone is then washed with one volume of absolute ethanol and dried over one volume of anhydrous ether yielding defatted bone powder. The defatted bone powder is then demineralized by four successive treatments with 10 volumes of 0.5 N HCl at 4°C for 40 min. Finally, neutralizing washes are done on the demineralized bone powder with a large volume of water.

### 2. Guanidine Extraction

Demineralized bone matrix thus prepared is extracted with 5 volumes of 4 M guanidine-HCl, 50mM Tris-HCl, pH 7.0 for 16 hr. at 4°C. The suspension is filtered. The insoluble material is collected and used to fabricate the matrix. The material is mostly collagenous in nature. It is devoid of osteogenic or chondrogenic activity.

### 3. Matrix Treatments

The major component of all bone matrices is Type-I collagen. In addition to collagen, demineralized bone extracted as disclosed above includes non-collagenous proteins which may account for 5% of its mass. In a xenogenic matrix, these noncollagenous components may present themselves as potent antigens, and may constitute immunogenic and/or inhibitory components. These components also may inhibit osteogenesis in allogenic implants by interfering with the developmental cascade of bone differentiation. It has been discovered that treatment of the matrix particles with a collagen fibril-modifying agent extracts potentially unwanted components from the matrix, and alters the surface structure of the matrix material. Useful agents include acids, organic solvents or heated aqueous media. Various treatments are described below. A detailed physical analysis of the effect these fibril-modifying agents have on demineralized, quanidine-extracted bone collagen particles is disclosed in PCT WO 90/10018, published 7-SEP-90.

After contact with the fibril-modifying agent, the treated matrix is washed to remove any extracted components, following a form of the procedure set forth below:
1. Suspend in TBS (Tris-buffered saline) lg/200 ml and stir at 4°C for 2 hrs; or in 6 M urea, 50 mM Tris-HCl, 500 mM NaCl, pH 7.0 (UTBS) or water and stir at room temperature (RT) for 30 minutes (sufficient time to neutralize the pH);
2. Centrifuge and repeat wash step; and
3. Centrifuge; discard supernatant; water wash residue; and then lyophilize.

### 3.1 Acid Treatments

### 1. Trifluoroacetic acid.

Trifluoroacetic acid is a strong non-oxidizing acid that is a known swelling agent for proteins, and which modifies collagen fibrils.

Bovine bone residue prepared as described above is sieved, and particles of the appropriate size are collected. These particles are extracted with various percentages (1.0% to 100%) of trifluoroacetic acid and water (v/v) at 0°C or room temperature for 1-2 hours with constant stirring. The treated matrix is filtered, lyophilized, or washed with water/salt and then lyophilized.

### 2. Hydrogen Fluoride.

Like trifluoroacetic acid, hydrogen fluoride is a strong acid and swelling agent, and also is capable of altering intraparticle surface structure. Hydrogen fluoride is also a known deglycosylating agent. As such, HF may function to increase the osteogenic activity of these matrices by removing the antigenic carbohydrate content of any glycoproteins still associated with the matrix after guanidine extraction.

Bovine bone residue prepared as described above is sieved, and particles of the appropriate size are collected. The sample is dried in vacuo over P₂O₅, transferred to the reaction vessel and exposed to anhydrous hydrogen fluoride (10-20 ml/g of matrix) by distillation onto the sample at -70°C. The vessel is allowed to warm to 0°C and the reaction mixture is stirred at this temperature for 120 minutes. After evaporation of the hydrogen fluoride in vacuo, the residue is dried thoroughly in vacuo over KOH pellets to remove any remaining traces of acid. Extent of deglycosylation can be determined from carbohydrate analysis of matrix samples taken before and after treatment with hydrogen fluoride, after washing the samples appropriately to remove non-covalently bound carbohydrates. SDS-extracted protein from HF-treated material is negative for carbohydrate as determined by Con A blotting.

The deglycosylated bone matrix is next washed twice in TBS (Tris-buffered saline) or UTBS, water-washed, and then lyophilized.

Other acid treatments are envisioned in addition to HF and TFA. TFA is a currently preferred acidifying reagent in these treatments because of its volatility. However, it is understood that other, potentially less caustic acids may be used, such as acetic or formic acid.

### 3.2 Solvent Treatment

### 1. Dichloromethane.

Dichloromethane (DCM) is an organic solvent capable of denaturing proteins without affecting their primary structure. This swelling agent is a common reagent in automated peptide synthesis, and is used in washing steps to remove components.

Bovine bone residue, prepared as described above, is sieved, and particles of the appropriate size are incubated in 100% DCM or, preferably, 99.9% DCM/0.1% TFA. The matrix is incubated with the swelling agent for one or two hours at 0°C or at room temperature. Alternatively, the matrix is treated with the agent at least three times with short washes (20 minutes each) with no incubation.

### 2. Acetonitrile.

Acetonitrile (ACN) is an organic solvent, capable of denaturing proteins without affecting their primary structure. It is a common reagent used in high-performance liquid chromatography, and is used to elute proteins from silica-based columns by perturbing hydrophobic interactions.

Bovine bone residue particles of the appropriate size, prepared as described above, are treated with 100% ACN (1.0 g/30 ml) or, preferably, 99.9% ACN/0.1% TFA at room temperature for 1-2 hours with constant stirring. The treated matrix is then water-washed, or washed with urea buffer, or 4 M NaCl and lyophilized. Alternatively, the ACN or ACN/TFA treated matrix may be lyophilized without wash.

### 3. Isopropanol.

Isopropanol is also an organic solvent capable of denaturing proteins without affecting their primary structure. It is a common reagent used to elute proteins from silica HPLC columns.

Bovine bone residue particles of the appropriate size prepared as described above are treated with 100% isopropanol (1.0 g/30 ml) or, preferably, in the presence of 0.1% TFA, at room temperature for 1-2 hours with constant stirring. The matrix is then water-washed or washed with urea buffer or 4 M NaCl before being lyophilized.

### 4. Chloroform

Chloroform also may be used to increase surface area of bone matrix like the reagents set forth above, either alone or acidified.

Treatment as set forth above is effective to assure that the material is free of pathogens prior to implantation.

### 3.3 Heat Treatment

The currently most preferred agent is a heated aqueous fibril-modifying medium such as water, to increase the matrix particle surface area and porosity. The currently most preferred aqueous medium is an acidic aqueous medium having a pH of less than about 4.5, e.g., within the range of pH 2 - pH 4. which may help to "swell" the collagen before heating. 0.1% acetic acid, which has a pH of about 3, currently is preferred. 0.1 M acetic acid also may be used.

Various amounts of delipidated, demineralized guanidine-extracted bone collagen are heated in the aqueous medium (1g matrix/30ml aqueous medium) under constant stirring in a water jacketed glass flask, and maintained at a given temperature for a predetermined period of time. Preferred treatment times are about one hour, although exposure times of between about 0.5 to two hours appear acceptable. The temperature employed is held constant at a temperature generally within the range of about 37°C to 75°C. The currently preferred heat treatment temperature is within the range of 45°C to 60°C.

After the heat treatment, the matrix is filtered, washed, lyophilized and used for implant. Where an acidic aqueous medium is used, the matrix also is preferably neutralized prior to washing and lyophilization. A currently preferred neutralization buffer is a 200mM sodium phosphate buffer, pH 7.0. To neutralize the matrix, the matrix preferably first is allowed to cool following thermal treatment, the acidic aqueous medium (e.g., 0.1% acetic acid) then is removed and replaced with the neutralization buffer and the matrix agitated for about 30 minutes. The neutralization buffer then may be removed and the matrix washed and lyophilized (see infra).

The matrix also may be treated to remove contaminating heavy metals, such as by exposing the matrix to a metal ion chelator. For example, following thermal treatment with 0.1% acetic acid, the matrix may be neutralized in a neutralization buffer containing EDTA (sodium ethylenediaminetetraacetic acid), e.g., 200 mM sodium phosphate, 5mM EDTA, pH 7.0. 5 mM EDTA provides about a 100-fold molar excess of chelator to residual heavy metals present in the most contaminated matrix tested to date. Subsequent washing of the matrix following neutralization appears to remove the bulk of the EDTA. EDTA treatment of matrix particles reduces the residual heavy metal content of all metals tested (Sb, As, Be, Cd, Cr, Cu, Co, Pb, Hg, Ni, Se, Ag, Zn, T1) to less than about 1 ppm. Bioassays with EDTA-treated matrices indicate that treatment with the metal ion chelator does not inhibit bone inducing activity.

The collagen matrix materials preferably take the form of a fine powder, insoluble in water, comprising nonadherent particles. It may be used simply by packing into the volume where new bone growth or sustained release is desired, held in place by surrounding tissue. Alternatively, the powder may be encapsulated in, e.g., a gelatin or polylactic acid coating, which is adsorbed readily by the body. The powder may be shaped to a volume of given dimensions and held in that shape by interadhering the particles using, for example, soluble, species-biocompatible collagen. The material may also be produced in sheet, rod, bead, or other macroscopic shapes.

Demineralized rat bone matrix used as an allogenic matrix in certain of the experiments disclosed herein, is prepared from several of the dehydrated diaphyseal shafts of rat femur and tibia as described herein to produce a bone particle size which passes through a 420 µm sieve. The bone particles are subjected to dissociative extraction with 4 M guanidine-HCl. Such treatment results in a complete loss of the inherent ability of the bone matrix to induce endochondral bone differentiation. The remaining insoluble material is used to fabricate the matrix. The material is mostly collagenous in nature, and upon implantation, does not induce cartilage and bone. All new preparations are tested for mineral content and osteogenic activity before use. The total loss of biological activity of bone matrix is restored when an active osteoinductive protein fraction or a pure osteoinductive protein preparation is reconstituted with the biologically inactive insoluble collagenous matrix.

### FABRICATION OF OSTEOGENIC DEVICE

The naturally sourced and recombinant protein as set forth above, and other constructs, can be combined and dispersed in a suitable matrix preparation using any of the methods described below. In general, 50-100 ng of active protein is combined with the inactive carrier matrix (e.g., 25 mg for rat bioassays). Greater amounts may be used for large implants.

### 1. Ethanol Precipitation

Matrix is added to osteogenic protein dissolved in guanidine-HCl. Samples are vortexed and incubated at a low temperature (e.g., 4°C). Samples are then further vortexed. Cold absolute ethanol (5 volumes) is added to the mixture which is then stirred and incubated, preferably for 30 minutes at -20°C. After centrifugation (microfuge, high speed) the supernatant is discarded. The reconstituted matrix is washed twice with cold concentrated ethanol in water (85% EtOH) and then lyophilized.

### 2. Acetonitrile Trifluoroacetic

### Acid Lyophilization

In this procedure, osteogenic protein in an acetonitrile trifluroacetic acid (ACN/TFA) solution is added to the carrier material. Samples are vigorously vortexed many times and then lyophilized. This method is currently preferred, and has been tested with osteogenic protein at varying concentrations and different levels of purity.

### 3. Urea Lyophilization

For those osteogenic proteins that are prepared in urea buffer, the protein is mixed with the matrix material, vortexed many times, and then lyophilized. The lyophilized material may be used "as is" for implants.

### 4. Buffered Saline Lyophilization

OP1 and OP2 preparations in physiological saline may also be vortexed with the matrix and lyophilized to produce osteogenically active material.

These procedures also can be used to adsorb other active therapeutic drugs, hormones, and various bioactive species to the matrix for sustained release purposes.

### BIOASSAY

The functioning of the various proteins and devices of this invention can be evaluated with an in vivo bioassay. Studies in rats show the osteogenic effect in an appropriate matrix to be dependent on the dose of osteogenic protein dispersed in the matrix. No activity is observed if the matrix is implanted alone. In vivo bioassays performed in the rat model also have shown that demineralized, guanidine-extracted xenogenic bone matrix materials of the type described in the literature are ineffective as a carrier, fail to induce bone, and produce an inflammatory and immunological response when implanted unless treated as disclosed above. In certain species (e.g., monkey) allogenic matrix materials also apparently are ineffective as carriers. The following sets forth various procedures for preparing osteogenic devices from the proteins and matrix materials prepared as set forth above, and for evaluating their osteogenic utility.

### A. Rat Model

### 1. Implantation

The bioassay for bone induction as described by Sampath and Reddi ((1983) Proc. Natl. Acad. Sci. USA 80 6591-6595), herein incorporated by reference, may be used to monitor endochondral bone differentiation activity. This assay consists of implanting test samples in subcutaneous sites in recipient rats under ether anesthesia. Male Long-Evans rats, aged 28-32 days, were used. A vertical incision (1 cm) is made under sterile conditions in the skin over the thoracic region, and a pocket is prepared by blunt dissection. Approximately 25 mg of the test sample is implanted deep into the pocket and the incision is closed with a metallic skin clip. The day of implantation is designated as day one of the experiment. Implants were removed on day 12. The heterotropic site allows for the study of bone induction without the possible ambiguities resulting from the use of orthotropic sites. As disclosed herein, both allogenic (rat bone matrix) and xenogenic (bovine bone matrix) implants were assayed.

### 2. Cellular Events

Successful implants exhibit a controlled progression through the stages of protein-induced endochondral bone development, including: (1) transient infiltration by polymorphonuclear leukocytes on day one; (2) mesenchymal cell migration and proliferation on days two and three; (3) chondrocyte appearance on days five and six; (4) cartilage matrix formation on day seven; (5) cartilage calcification on day eight; (6) vascular invasion, appearance of osteoblasts, and formation of new bone on days nine and ten; (7) appearance of osteoblastic and bone remodeling and dissolution of the implanted matrix on days twelve to eighteen; and (8) hematopoietic bone marrow differentiation in the ossicle on day twenty-one. The results show that the shape of the new bone conforms to the shape of the implanted matrix.

### 3. Histological Evaluation

Histological sectioning and staining is preferred to determine the extent of osteogenesis in the implants. Implants are fixed in Bouins Solution, embedded in paraffin, and cut into 6-8 µm sections. Staining with toluidine blue or hemotoxylin/eosin demonstrates clearly the ultimate development of endochondral bone. Twelve day implants are usually sufficient to determine whether the implants contain newly induced bone.

### 4. Biological Markers

Alkaline phosphatase activity may be used as a marker for osteogenesis. The enzyme activity may be determined spectrophotometrically after homogenization of the implant. The activity peaks at 9-10 days in vivo and thereafter slowly declines. Implants showing no bone development by histology have little or no alkaline phosphatase activity under these assay conditions. The assay is useful for quantitation and obtaining an estimate of bone formation quickly after the implants are removed from the rat. Alternatively, the amount of bone formation can be determined by measuring the calcium content of the implant.

The invention may be embodied in other specific forms. The present embodiments are therefore to be considered in all respects as illustrative and not restrictive, the scope of the invention being indicated by the appended claims rather than by the foregoing description.

### SEQUENCE LISTING

### (1) GENERAL INFORMATION:

(i) APPLICANT: OPPERMANN, HERNANN
   OZKAYNAK, ENGIN
   RUEGER, DAVID C.
   KUBERASAMPATH, THANGAVEL
(ii) TITLE OF INVENTION: OSTEOGENIC DEVICES
(iii) NUMBER OF SEQUENCES: 9
(iv) CORRESPONDENCE ADDRESS:
   (A) ADDRESSEE: TESTA, HURVITZ & THIBEAULT
   (B) STREET: 53 STATE STREET
   (C) CITY: BOSTON
   (D) STATE: MASSACHUSETTS
   (E) COUNTRY: U.S.A.
   (F) ZIP: 02109
(v) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25
(vi) CURRENT APPLICATION DATA:
   (A) APPLICATION NUMBER:
   (B) FILING DATE:
   (C) CLASSIFICATION:
(viii) ATTORNEY/AGENT INFORHATION:
   (A) NAME: PITCHER, EDMUND R.
   (B) REGISTRATION NUMBER: 27,829
   (C) REFERENCE/DOCKET NUMBER: CRR056PC
(ix) TELECOMMUNICATION INFORMATION:
   (A) TELEPHONE: 617/248-7000
   (B) TELEFAX: 617/248-7100

### (2) INFORHATION FOR SEQ ID NO:1:

(i) SEQUENCE CHARACTERISTICS:
   (A) LENGTH: 1822 base pairs
   (B) TYPE: nucleic acid
   (C) STRANDEDNESS: single
   (D) TOPOLOGY: linear
(ii) MOLECULE TYPE: cDNA
(iii) HYPOTHETICAL: NO
(iv) ANTI-SENSE: NO
(vi) ORIGINAL SOURCE:
   (A) ORGANISM: HOMO SAPIENS
   (F) TISSUE TYPE: HIPPOCAMPUS
(ix) FEATURE:
   (A) NAME/KEY: CDS
   (B) LOCATION: 49..1341
   (C) IDENTIFICATION METHOD: experimental
   (D) OTHER INFORMATION: /function= "OSTEOGENIC PROTEIN"
      /product= "h0P1-PP"
      /evidence= EXPERIMENTAL
      /standard_name= "hOP1"
(xi) SEQUENCE DESCRIPTION: SEQ ID NO:1:

### (2) INFORMATION FOR SEQ ID NO:2:

(i) SEQUENCE CHARACTERISTICS:
   (A) LENGTH: 431 amino acids
   (B) TYPE: amino acid
   (D) TOPOLOGY: linear
(ii) MOLECULE TYPE: protein
(ix) FEATURE:
   (D) OTHER INFORMATION: /Product="h0P1-PP"
(xi) SEQUENCE DESCRIPTION: SEQ ID NO:2:
   Met His Val Arg Ser Leu Arg Ala Ala Ala Pro His Ser Phe Val Ala
    1               5                  10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
           260                 265                 270
   Lys Gln Pro Phe Met Val Ala Phe Phe Lys Ala Thr Glu Val His Phe
           275                 280                 285
   Arg Ser Ile Arg Ser Thr Gly Ser Lys Gln Arg Ser Gln Asn Arg Ser
       290                 295                 300
   Lys Thr Pro Lys Asn Gln Glu Ala Leu Arg Met Ala Asn Val Ala Glu
   305                 310                 315                 320
   Asn Ser Ser Ser Asp Gln Arg Gln Ala Cys Lys Lys His Glu Leu Tyr
                   325                 330                 335
   Val Ser Phe Arg Asp Leu Gly Trp Gln Asp Trp Ile Ile Ala Pro Glu
               340                 345                 350
   Gly Tyr Ala Ala Tyr Tyr Cys Glu Gly Glu Cys Ala Phe Pro Leu Asn
           355                 360                 365
   Ser Tyr Met Asn Ala Thr Asn His Ala Ile Val Gln Thr Leu Val His
       370                 375                 380
   Phe Ile Asn Pro Glu Thr Val Pro Lys Pro Cys Cys Ala Pro Thr Gln
   385                 390                 395                 400
   Leu Asn Ala Ile Ser Val Leu Tyr Phe Asp Asp Ser Ser Asn Val Ile
                   405                 410                 415
   Leu Lys Lys Tyr Arg Asn Met Val Val Arg Ala Cys Gly Cys His
               420                 425                 430

### (2) INFORMATION FOR SEQ ID NO:3:

(i) SEQUENCE CHARACTERISTICS:
   (A) LENGTH: 1929 base pairs
   (B) TYPE: nucleic acid
   (C) STRANDEDNESS: single
   (D TOPOLOGY: linear
(ii) MOLECULE TYPE: cDNA
(ix) FEATURE:
   (A) NAME/KEY: CDS
   (B) LOCATION: 103..1293
   (D) OTHER INFORMATION: /function= "osteogenic protein"
      /product= "mOP2-PP"
      /note= "mOP2 cDNA"
(xi) SEQUENCE DESCRIPTION: SEQ ID NO:3:
   GAATTCCGCT GCCAGGCACA GGTGCGCCGT CTGGTCCTCC CCGTCTGGCG TCAGCCGAGC        60
   CCGACCAGCT ACCAGTGGAT GCGCGCCGGC TGAAAGTCCG AG ATG GCT ATG CGT      114
                                                  Met Ala Met Arg
                                                   1
   CCC GGG CCA CTC TGG CTA TTG GGC CTT GCT CTG TGC GCG CTG GGA GGC     162
   Pro Gly Pro Leu Trp Leu Leu Gly Leu Ala Leu Cys Ala Leu Gly Gly
    5                  10                  15                  20
   GGC CAC GGT CCC GGT CCC CCG CAC ACC TGT CCC CAG CGT CGC CTG GGA     210
   Gly His Gly Pro Gly Pro Pro His Thr Cys Pro Gln Arg Arg Leu Gly
                   25                  30                  35
   GCG CGC GAC CGG GAC ATG CAG CGT GAA ATC CTG CCG GTG CTC GGG CTA     258
   Ala Arg Asp Arg Asp Met Gln Arg Glu Ile Leu Pro Val Leu Gly Leu
               40                  45                  50
   CCG GGA CGC CCC GAC CCC GTG CAC AAC CCG CCG CTG CCC GGC ACG CAG     306
   Pro Gly Arg Pro Asp Pro Val His Asn Pro Pro Leu Pro Gly Thr Gln
           55                  60                  65
   CGT GCG CCC CTC TTC ATG TTG GAC CTA TAC CAC GCC ATG ACC GAT GAC     354
   Arg Ala Pro Leu Phe Met Leu Asp Leu Tyr His Ala Met Thr Asp Asp
       70                  75                  80
   GAC GAC GGC GGG CCA CCA CAG GCT CAC TTA GGC CGT GCC GAC CTG GTC     402
   Asp Asp Gly Gly Pro Pro Gln Ala His Leu Gly Arg Ala Asp Leu Val
   85                  90                  95                  100
   ATG AGC TTC GTC AAC ATG GTG GAA CGC GAC CGT ACC CTG GGC TAC CAG     450
   Met Ser Phe Val Asn Met Val Glu Arg Asp Arg Thr Leu Gly Tyr Gln
                   105                 110                 115
   GAG CCA CAC TGG AAG GAA TTC CAC TTT GAC CTA ACC CAG ATC CCT GCT     498
   Glu Pro His Trp Lys Glu Phe His Phe Asp Leu Thr Gln Ile Pro Ala
               120                 125                 130
   GGG GAG GCT GTC ACA GCT GCT GAG TTC CGG ATC TAC AAA GAA CCC AGC     546
   Gly Glu Ala Val Thr Ala Ala Glu Phe Arg Ile Tyr Lys Glu Pro Ser
           135                 140                 145
   ACC CAC CCG CTC AAC ACA ACC CTC CAC ATC AGC ATG TTC GAA GTG GTC     594
   Thr His Pro Leu Asn Thr Thr Leu His Ile Ser Met Phe Glu Val Val
       150                 155                 160
   CAA GAG CAC TCC AAC AGG GAG TCT GAC TTG TTC TTT TTG GAT CTT CAG     642
   Gln Glu His Ser Asn Arg Glu Ser Asp Leu Phe Phe Leu Asp Leu Gln
   165                 170                 175                 180
   ACG CTC CGA TCT GGG GAC GAG GGC TGG CTG GTG CTG GAC ATC ACA GCA     690
   Thr Leu Arg Ser Gly Asp Glu Gly Trp Leu Val Leu Asp Ile Thr Ala
                   185                 190                 195
   GCC AGT GAC CGA TGG CTG CTG AAC CAT CAC AAG GAC CTG GGA CTC CGC     738
   Ala Ser Asp Arg Trp Leu Leu Asn His His Lys Asp Leu Gly Leu Arg
               200                 205                 210
   CTC TAT GTG GAA ACC GCG GAT GGG CAC AGC ATG GAT CCT GGC CTG GCT     786
   Leu Tyr Val Glu Thr Ala Asp Gly His Ser Met Asp Pro Gly Leu Ala
           215                 220                 225
   GGT CTG CTT GGA CGA CAA GCA CCA CGC TCC AGA CAG CCT TTC ATG GTA     834
   Gly Leu Leu Gly Arg Gln Ala Pro Arg Ser Arg Gln Pro Phe Met Val
       230                 235                 240
   ACC TTC TTC AGG GCC AGC CAG AGT CCT GTG CGG GCC CCT CGG GCA GCG     882
   Thr Phe Phe Arg Ala Ser Gln Ser Pro Val Arg Ala Pro Arg Ala Ala
   245                 250                 255                 260
   AGA CCA CTG AAG AGG AGG CAG CCA AAG AAA ACG AAC GAG CTT CCG CAC     930
   Arg Pro Leu Lys Arg Arg Gln Pro Lys Lys Thr Asn Glu Leu Pro His
                   265                 270                 275
   CCC AAC AAA CTC CCA GGG ATC TTT GAT GAT GGC CAC GGT TCC CGC GGC     978
   Pro Asn Lys Leu Pro Gly Ile Phe Asp Asp Gly His Gly Ser Arg Gly
               280                 285                 290
   AGA GAG GTT TGC CGC AGG CAT GAG CTC TAC GTC AGA TTC CGT GAC CTT    1026
   Arg Glu Val Cys Arg Arg His Glu Leu Tyr Val Arg Phe Arg Asp Leu
           295                 300                 305
   GGC TGG CTG GAC TGG GTC ATC GCC CCC CAG GGC TAC TCT GCC TAT TAC    1074
   Gly Trp Leu Asp Trp Val Ile Ala Pro Gln Gly Tyr Ser Ala Tyr Tyr
       310                 315                 320
   TGT GAG GGG GAG TGT GCT TTC CCA CTG GAC TCC TGT ATG AAC GCC ACC    1122
   Cys Glu Gly Glu Cys Ala Phe Pro Leu Asp Ser Cys Met Asn Ala Thr
   325                 330                 335                 340
   AAC CAT GCC ATC TTG CAG TCT CTG GTG CAC CTG ATG AAG CCA GAT GTT    1170
   Asn His Ala Ile Leu Gln Ser Leu Val His Leu Met Lys Pro Asp Val
                   345                 350                 355
   GTC CCC AAG GCA TGC TGT GCA CCC ACC AAA CTG AGT GCC ACC TCT GTG    1218
   Val Pro Lys Ala Cys Cys Ala Pro Thr Lys Leu Ser Ala Thr Ser Val
               360                 365                 370
   CTG TAC TAT GAC AGC AGC AAC AAT GTC ATC CTG CGT AAA CAC CGT AAC    1266
   Leu Tyr Tyr Asp Ser Ser Asn Asn Val Ile Leu Arg Lys His Arg Asn
           375                 380                 385
   ATG GTG GTC AAG GCC TGT GGC TGC CAC TGAGGCCCCG CCCAGCATCC          1313
   Met Val Val Lys Ala Cys Gly Cys His
       390                 395
   TGCTTCTACT ACCTTACCAT CTGGCCGGGC CCCTCTCCAG AGGCAGAAAC CCTTCTATGT  1373
   TATCATAGCT CAGACAGGGG CAATGGGAGG CCCTTCACTT CCCCTGGCCA CTTCCTGCTA      1433
   AAATTCTGGT CTTTCCCAGT TCCTCTGTCC TTCATGGGGT TTCGGGGCTA TCACCCCGCC      1493
   CTCTCCATCC TCCTACCCCA AGCATAGACT GAATGCACAC AGCATCCCAG AGCTATGCTA      1553
   ACTGAGAGGT CTGGGGTCAG CACTGAAGGC CCACATGAGG AAGACTGATC CTTGGCCATC      1613
   CTCAGCCCAC AATGGCAAAT TCTGGATGGT CTAAGAAGCC CTGGAATTCT AAACTAGATG      1673
   ATCTGGGCTC TCTGCACCAT TCATTGTGGC AGTTGGGACA TTTTTAGGTA TAACAGACAC      1733
   ATACACTTAG ATCAATGCAT CGCTGTACTC CTTGAAATCA GAGCTAGCTT GTTAGAAAAA      1793
   GAATCAGAGC CAGGTATAGC GGTGCATGTC ATTAATCCCA GCGCTAAAGA GACAGAGACA      1853
   GGAGAATCTC TGTGAGTTCA AGGCCACATA GAAAGAGCCT GTCTCGGGAG CAGGAAAAAA      1913
   AAAAAAAACG GAATTC                                                      1929

### (2) INFORHATION FOR SEQ ID NO:4:

(i) SEQUENCE CHARACTERISTICS:
   (A) LENGTH: 397 amino acids
   (B) TYPE: amino acid
   (D) TOPOLOGY: linear
(ii) MOLECULE TYPE: protein
(ix) FEATURE:
   (D) OTHER INFORMATION: /Product= "mOP2-PP"
(xi) SEQUENCE DESCRIPTION: SEQ ID NO:4:

### (2) INFORMATION FOR SEQ ID NO:5:

(i) SEQUENCE CHARACTERISTICS:
   (A) LENGTH: 1941 base pairs
   (B) TYPE: nucleic acid
   (C) STRANDEDNESS: single
   (D) TOPOLOGY: linear
(ii) MOLECULE TYPE: cDNA
(vi) ORIGINAL SOURCE:
   (A) ORGANISM: HOMO SAPIENS
   (F) TISSUE TYPE: HIPPOCAMPUS
(ix) FEATURE:
   (A) NAME/KEY: CDS
   (B) LOCATION: 507..1703
   (D) OTHER INFORMATION: /function= "OSTEOGENIC PROTEIN"
      /product= "hOP2-PP"
      /note= "hOP2 (CDNA)"
   (xi) SEQUENCE DESCRIPTION: SEQ ID NO:5:
   GGAATTCCGG CCACAGTGGC GCCGGCAGAG CAGGAGTGGC TGGAGGAGCT GTGGTTGGAG      60
   CAGGAGGTGG CACGGCAGGG CTGGAGGGCT CCCTATGAGT GGCGGAGACG GCCCAGGAGG     120
   CGCTGGAGCA ACAGCTCCCA CACCGCACCA AGCGGTGGCT GCAGGAGCTC GCCCATCGCC     180
   CCTGCGCTGC TCGGACCGCG GCCACAGCCG GACTGGCGGG TACGGCGGCG ACAGAGGCAT     240
   TGGCCGAGAG TCCCAGTCCG CAGAGTAGCC CCGGCCTCGA GGCGGTGGCG TCCCGGTCCT     300
   CTCCGTCCAG GAGCCAGGAC AGGTGTCGCG CGGCGGGGCT CCAGGGACCG CGCCTGAGGC     360
   CGGCTGCCCG CCCGTCCCGC CCCGCCCCGC CGCCCGCCGC CCGCCGAGCC CAGCCTCCTT     420
   GCCGTCGGGG CGTCCCCAGG CCCTGGGTCG GCCGCGGAGC CGATGCGCGC CCGCTGAGCG     480
   CCCCAGCTGA GCGCCCCCGG CCTGCC ATG ACC GCG CTC CCC GGC CCG CTC TGG     533
                                Met Thr Ala Leu Pro Gly Pro Leu Trp
                                 1               5
   CTC CTG GGC CTG GCG CTA TGC GCG CTG GGC GGG GGC GGC CCC GGC CTG      581
   Leu Leu Gly Leu Ala Leu Cys Ala Leu Gly Gly Gly Gly Pro Gly Leu
    10                  15                  20                  25
   CGA CCC CCG CCC GGC TGT CCC CAG CGA CGT CTG GGC GCG CGC GAC CGG      629
   Arg Pro Pro Pro Gly Cys Pro Gln Arg Arg Leu Gly Ala Arg Asp Arg
                   30                  35                  40
   GAC GTG CAG CGC GAG ATC CTG GCG GTG CTC GGG CTG CCT GGG CGG CCC      677
   Asp Val Gln Arg Glu Ile Leu Ala Val Leu Gly Leu Pro Gly Arg Pro
               45                  50                  55
   CGG CCC CGC GCG CCA CCC GCC GCC TCC CGG CTG CCC GCG TCC GCG CCG      725
   Arg Pro Arg Ala Pro Pro Ala Ala Ser Arg Leu Pro Ala Ser Ala Pro
           60                  65                  70
   CTC TTC ATG CTG GAC CTG TAC CAC CGC ATG GCC GGC GAC GAC GAC GAG      773
   Leu Phe Met Leu Asp Leu Tyr His Arg Met Ala Gly Asp Asp Asp Glu
       75                  80                  85
   GAC GGC GCC GCG GAG GCC CTG GGC CGC GCC GAC CTG GTC ATG AGC TTC      821
   Asp Gly Ala Ala Glu Ala Leu Gly Arg Ala Asp Leu Val Met Ser Phe
   90                  95                  100                 105
   GTT AAC ATG GTG GAG CGA GAC CGT GCC CTG GGC CAC CAG GAG CCC CAT      869
   Val Asn Met Val Glu Arg Asp Arg Ala Leu Gly His Gln Glu Pro His
                   110                 115                 120
   TGG AAG GAG TTC CGC TTT GAC CTG ACC CAG ATC CCG GCT GGG GAG GCG      917
   Trp Lys Glu Phe Arg Phe Asp Leu Thr Gln Ile Pro Ala Gly Glu Ala
               125                 130                 135
   GTC ACA GCT GCG GAG TTC CGG ATT TAC AAG GTG CCC AGC ATC CAC CTG      965
   Val Thr Ala Ala Glu Phe Arg Ile Tyr Lys Val Pro Ser Ile His Leu
           140                 145                 150
   CTC AAC AGG ACC CTC CAC GTC AGC ATG TTC CAG GTG GTC CAG GAG CAG     1013
   Leu Asn Arg Thr Leu His Val Ser Met Phe Gln Val Val Gln Glu Gln
       155                 160                 165
   TCC AAC AGG GAG TCT GAC TTG TTC TTT TTG GAT CTT CAG ACG CTC CGA     1061
   Ser Asn Arg Glu Ser Asp Leu Phe Phe Leu Asp Leu Gln Thr Leu Arg
   170                 175                 180                 185
   GCT GGA GAC GAG GGC TGG CTG GTG CTG GAT GTC ACA GCA GCC AGT GAC     1109
   Ala Gly Asp Glu Gly Trp Leu Val Leu Asp Val Thr Ala Ala Ser Asp
                   190                 195                 200
   TGC TGG TTG CTG AAG CGT CAC AAG GAC CTG GGA CTC CGC CTC TAT GTG     1157
   Cys Trp Leu Leu Lys Arg His Lys Asp Leu Gly Leu Arg Leu Tyr Val
               205                 210                 215
   GAG ACT GAG GAC GGG CAC AGC GTG GAT CCT GGC CTG GCC GGC CTG CTG     1205
   Glu Thr Glu Asp Gly His Ser Val Asp Pro Gly Leu Ala Gly Leu Leu
           220                 225                 230
   GGT CAA CGG GCC CCA CGC TCC CAA CAG CCT TTC GTG GTC ACT TTC TTC     1253
   Gly Gln Arg Ala Pro Arg Ser Gln Gln Pro Phe Val Val Thr Phe Phe
       235                     240                     245
   AGG GCC AGT CCG AGT CCC ATC CGC ACC CCT CGG GCA GTG AGG CCA CTG       1301
   Arg Ala Ser Pro Ser Pro Ile Arg Thr Pro Arg Ala Val Arg Pro Leu
   250                 255                 260                 265
   AGG AGG AGG CAG CCG AAG AAA AGC AAC GAG CTG CCG CAG GCC AAC CGA       1349
   Arg Arg Arg Gln Pro Lys Lys Ser Asn Glu Leu Pro Gln Ala Asn Arg
                   270                 275                 280
   CTC CCA GGG ATC TTT GAT GAC GTC CAC GGC TCC CAC GGC CGG CAG GTC       1397
   Leu Pro Gly Ile Phe Asp Asp Val His Gly Ser His Gly Arg Gln Val
               285                 290                 295
   TGC CGT CGG CAC GAG CTC TAC GTC AGC TTC CAG GAC CTC GGC TGG CTG       1445
   Cys Arg Arg His Glu Leu Tyr Val Ser Phe Gln Asp Leu Gly Trp Leu
           300                 305                 310
   GAC TGG GTC ATC GCT CCC CAA GGC TAC TCG GCC TAT TAC TGT GAG GGG       1493
   Asp Trp Val Ile Ala Pro Gln Gly Tyr Ser Ala Tyr Tyr Cys Glu Gly
       315                 320                 325
   GAG TGC TCC TTC CCA CTG GAC TCC TGC ATG AAT GCC ACC AAC CAC GCC       1541
   Glu Cys Ser Phe Pro Leu Asp Ser Cys Met Asn Ala Thr Asn His Ala
   330                 335                 340                 345
   ATC CTG CAG TCC CTG GTG CAC CTG ATG AAG CCA AAC GCA GTC CCC AAG       1589
   Ile Leu Gln Ser Leu Val His Leu Met Lys Pro Asn Ala Val Pro Lys
                   350                 355                 360
   GCG TGC TGT GCA CCC ACC AAG CTG AGC GCC ACC TCT GTG CTC TAC TAT       1637
   Ala Cys Cys Ala Pro Thr Lys Leu Ser Ala Thr Ser Val Leu Tyr Tyr
               365                 370                 375
   GAC AGC AGC AAC AAC GTC ATC CTG CGC AAA GCC CGC AAC ATG GTG GTC       1685
   Asp Ser Ser Asn Asn Val Ile Leu Arg Lys Ala Arg Asn Met Val Val
           380                 385                 390
   AAG GCC TGC GGC TGC CAC TGAGTCAGCC CGCCCAGCCC TACTGCAGCA             1733
   Lys Ala Cys Gly Cys His
       395
   ATTCACTGGC CGTCGTTTTA CAACGTGTGA CTGGGAAAAC CCTGGCGTTA CCCAACTTAA   1793
   TCGCCTTGCA GCACATCCCC CTTTCGCCAG CTGGCTAATA GCGAAGAGGC CCCGCACCGA   1853
   TCGCCCTTCC CAACAGTTGC GCCCCAGTGA ATGGCGAATG GCAAATTGTA AGCGTTAATA   1913
   TTTTGTTAAA ATTCGCGTTA AATTTTTT                                       1941

### (2) INFORMATION FOR SEQ ID NO:6:

(i) SEQUENCE CHARACTERISTICS:
   (A) LENGTH: 399 amino acids
   (B) TYPE: amino acid
   (D) TOPOLOGY: linear
(ii) MOLECULE TYPE: protein
(ix) FEATURE:
   (D) OTHER INFORMATION: /product= "hOP2-PP"
(xi) SEQUENCE DESCRIPTION: SEQ ID NO:6:
   Met Thr Ala Leu Pro Gly Pro Leu Trp Leu Leu Gly Leu Ala Leu Cys
    1               5                  10                  15
   Ala Leu Gly Gly Gly Gly Pro Gly Leu Arg Pro Pro Pro Gly Cys Pro
               20                  25                  30
   Gln Arg Arg Leu Gly Ala Arg Asp Arg Asp Val Gln Arg Glu Ile Leu
           35                  40                  45
   Ala Val Leu Gly Leu Pro Gly Arg Pro Arg Pro Arg Ala Pro Pro Ala
       50                  55                  60
   Ala Ser Arg Leu Pro Ala Ser Ala Pro Leu Phe Met Leu Asp Leu Tyr
   65                  70                  75                  80
   His Arg Met Ala Gly Asp Asp Asp Glu Asp Gly Ala Ala Glu Ala Leu
                   85                  90                  95
   Gly Arg Ala Asp Leu Val Met Ser Phe Val Asn Met Val Glu Arg Asp
               100                 105                 110
   Arg Ala Leu Gly His Gln Glu Pro His Trp Lys Glu Phe Arg Phe Asp
           115                 120                 125
   Leu Thr Gln Ile Pro Ala Gly Glu Ala Val Thr Ala Ala Glu Phe Arg
       130                 135                 140
   Ile Tyr Lys Val Pro Ser Ile His Leu Leu Asn Arg Thr Leu His Val
   145                 150                 155                 160
   Ser Met Phe Gln Val Val Gln Glu Gln Ser Asn Arg Glu Ser Asp Leu
                   165                 170                 175
   Phe Phe Leu Asp Leu Gln Thr Leu Arg Ala Gly Asp Glu Gly Trp Leu
               180                 185                 190
   Val Leu Asp Val Thr Ala Ala Ser Asp Cys Trp Leu Leu Lys Arg His
           195                 200                 205
   Lys Asp Leu Gly Leu Arg Leu Tyr Val Glu Thr Glu Asp Gly His Ser
       210                 215                 220
   Val Asp Pro Gly Leu Ala Gly Leu Leu Gly Gln Arg Ala Pro Arg Ser
   225                 230                 235                 240
   Gln Gln Pro Phe Val Val Thr Phe Phe Arg Ala Ser Pro Ser Pro Ile
                   245                 250                 255
   Arg Thr Pro Arg Ala Val Arg Pro Leu Arg Arg Arg Gln Pro Lys Lys
               260                 265                 270
   Ser Asn Glu Leu Pro Gln Ala Asn Arg Leu Pro Gly Ile Phe Asp Asp
           275                 280                 285
   Val His Gly Ser His Gly Arg Gln Val Cys Arg Arg His Glu Leu Tyr
       290                 295                 300
   Val Ser Phe Gln Asp Leu Gly Trp Leu Asp Trp Val Ile Ala Pro Gln
   305                 310                 315                 320
   Gly Tyr Ser Ala Tyr Tyr Cys Glu Gly Glu Cys Ser Phe Pro Leu Asp
                   325                 330                 335
   Ser Cys Met Asn Ala Thr Asn His Ala Ile Leu Gln Ser Leu Val His
               340                 345                 350
   Leu Met Lys Pro Asn Ala Val Pro Lys Ala Cys Cys Ala Pro Thr Lys
           355                 360                 365
   Leu Ser Ala Thr Ser Val Leu Tyr Tyr Asp Ser Ser Asn Asn Val Ile
       370                 375                 380
   Leu Arg Lys Ala Arg Asn Met Val Val Lys Ala Cys Gly Cys His
   385                 390                 395

### (2) INFORMATION FOR SEQ ID NO:7:

(i) SEQUENCE CHARACTERISTICS:
   (A) LENGTH: 102 amino acids
   (B) TYPE: amino acid
   (D) TOPOLOGY: linear
(ii) MOLECULE TYPE: protein
(ix) FEATURE:
   (A) NAME/KEY: Protein
   (B) LOCATION: 1..102
   (D) OTHER INFORMATION: /label= OPX
      /note= "VHEREIN EACH XAA IS INDEPENDENTLY
      SELECTED FROM A GROUP OF ONE OR MORE
      SPECIFIED AMINO ACIDS AS DEFINED IN THE
      SPECIFICATION
(xi) SEQUENCE DESCRIPTION: SEQ ID NO:7:
   Cys Xaa Xaa His Glu Leu Tyr Val Xaa Phe Xaa Asp Leu Gly Trp Xaa
   1               5                   10                  15
   Asp Trp Xaa Ile Ala Pro Xaa Gly Tyr Xaa Ala Tyr Tyr Cys Glu Gly
               20                  25                  30
   Glu Cys Xaa Phe Pro Leu Xaa Ser Xaa Met Asn Ala Thr Asn His Ala
           35                  40                  45
   Ile Xaa Gln Xaa Leu Val His Xaa Xaa Xaa Pro Xaa Xaa Val Pro Lys
       50                  55                  60
   Xaa Cys Cys Ala Pro Thr Xaa Leu Xaa Ala Xaa Ser Val Leu Tyr Xaa
   65                  70                  75                      80
   Asp Xaa Ser Xaa Asn Val Xaa Leu Xaa Lys Xaa Arg Asn Met Val Val
                   85                  90                  95
   Xaa Ala Cys Gly Cys His
               100

### (2) INFORMATION FOR SEQ ID NO:8:

(i) SEQUENCE CHARACTERISTICS:
   (A) LENGTH: 97 amino acids
   (B) TYPE: amino acid
   (D) TOPOLOGY: linear
(ii) MOLECULE TYPE: protein
(ix) FEATURE:
   (A) NAME/KEY: Protein
   (B) LOCATION: 1..97
   (D) OTHER INFORMATION: /label= OPX-7C
      /note= "WHEREIN EACH XAA INDEPENDENTLY INDICATES
      ONE OF THE 20 NATURALLY-OCCURRING L-ISOMER,
      A-AMINO ACIDS, OR A DERIVATIVE THEREOF."
(xi) SEQUENCE DESCRIPTION: SEQ ID NO:8:
   Xaa Xaa Xaa Xaa Xaa Xaa Xaa Xaa Xaa Xaa Xaa Xaa Xaa Xaa Xaa Xaa
   1               5                   10                  15
   Xaa Xaa Xaa Xaa Xaa Xaa Xaa Xaa Cys Xaa Xaa Xaa Cys Xaa Xaa Xaa
               20                  25                  30
   Xaa Xaa Xaa Cys Xaa Xaa Xaa Xaa Xaa Xaa Xaa Xaa Xaa Xaa Xaa Xaa
           35                  40                  45
   Xaa Xaa Xaa Xaa Xaa Xaa Xaa Xaa Xaa Xaa Xaa Xaa Cys Cys Xaa Xaa
       50                  55                  60
   Xaa Xaa Xaa Xaa Xaa Xaa Xaa Xaa Xaa Xaa Xaa Xaa Xaa Xaa Xaa Xaa
   65 70 75 80
   Xaa Xaa Xaa Xaa Xaa Xaa Xaa Xaa Xaa Xaa Xaa Xaa Xaa Cys Xaa Cys
   85 90 95
   Xaa

### (2) INFORMATION FOR SEQ ID NO:9:

(i) SEQUENCE CHARACTERISTICS:
   (A) LENGTH: 102 amino acids
   (B) TYPE: amino acid
   (D) TOPOLOGY: linear
(ii) MOLECULE TYPE: protein
(ix) FEATURE:
   (A) NAME/KEY: Protein
   (B) LOCATION: 1..102
   (D) OTHER INFORMATION: /label= OPX-8C
      /note= "WHEREIN EACH XAA INDEPENDENTLY
      INDICATES ONE OF THE 20
      NATURALLY-OCCURRING L-ISOMER A-AMINO
      ACIDS, OR A DERIVATIVE THEREOF."
(xi) SEQUENCE DESCRIPTION: SEQ ID NO:9:
   Cys Xaa Xaa Xaa Xaa Xaa Xaa Xaa Xaa Xaa Xaa Xaa Xaa Xaa Xaa Xaa
   1 5 10 15
   Xaa Xaa Xaa Xaa Xaa Xaa Xaa Xaa Xaa Xaa Xaa Xaa Xaa Cys Xaa Xaa
   20 25 30
   Xaa Cys Xaa Xaa Xaa Xaa Xaa Xaa Cys Xaa Xaa Xaa Xaa Xaa Xaa Xaa
   35 40 45
   Xaa Xaa Xaa Xaa Xaa Xaa Xaa Xaa Xaa Xaa Xaa Xaa Xaa Xaa Xaa Xaa
   50 55 60
   Xaa Cys Cys Xaa Xaa Xaa Xaa Xaa Xaa Xaa Xaa Xaa Xaa Xaa Xaa Xaa
   65 70 75 80
   Xaa Xaa Xaa Xaa Xaa Xaa Xaa Xaa Xaa Xaa Xaa Xaa Xaa Xaa Xaa Xaa
   85 90 95
   Xaa Xaa Cys Xaa Cys Xaa
   100

## Claims

1. A polypeptide chain comprising an amino acid sequence described by residues 303-399 of Seq. ID No. 5.

2. The polypeptide chain of claim 1 comprising an amino acid sequence described by
(a) residues 298-399 of Seq. ID No. 5; or
(b) residues 267-399 of Seq. ID No. 5; or
(c) residues 264-399 of Seq. ID No. 5; or
(d) residues 240-399 of Seq. ID No. 5, or
(e) residues 1-399 of Seq. ID No. 5.

3. A polypeptide chain comprising an amino acid sequence described by residues of 301-397 of Seq. ID No. 3.

4. The polypeptide chain of claim 3 comprising an amino acid sequence described by (a) residues 296-397 of Seq. ID No.3; or
(b) residues 259-397 of Seq. ID No. 3; or
(c) residues 1-397 of Seq. ID No. 3.

5. A polypeptide chain useful as a subunit of a dimeric osteogenic protein comprising a pair of disulphide-bonded polypeptide chains, said polypeptide chain comprising an amino acid sequence which either:
(a) shares the conserved six cysteine skeleton depicted in Fig. 2.1 to 2.3 from Leu (position 43) to His (position 139) of hOP1 and further comprising an additional cysteine residue in said six cysteine skeleton, or
(b) shares the conserved seven cysteine skeleton depicted in Fig. 2.1 to 2.3 from Cys (position 38) to His (position 139) of hOP1 and further comprises an additional cysteine residue therein,
such that the dimeric osteogenic protein comprising said polypeptide chain has a conformation capable of inducing endochoncrai bore formation when implanted in a mammal in association with a matrix.

6. The polypeptide chain of claim 5 wherein said amino acid sequence comprises:
(a) residues 261-399 of Seq. ID No. 5; or
(b) residues 301-397 of Seq. ID No. 3; or
(c) residues 259-397 of Seq. ID No. 3; or
(d) residues 298-399 of Seq. ID No. 5.

7. A dimeric osteogenic protein capable of inducing endochondral bone formation in a mammal when implanted in said mammal in association with a matrix, said protein comprising a pair of disulphide-bonded polypeptide chains constituting a dimeric species, wherein each of said polypeptide chains is the polypeptide chain of claim 5.

8. The polypeptide chain of any one of claims 1 to 6 produced by expression of recombinant DNA in host cell, e.g. a prokaryotic host cell or a mammalian host cell.

9. The polypeptide chain of claim 8 wherein said host cell is selected from *E. coli,* CHO, COS, BSC, *Saccharomyces* or myeloma host cells.

10. The polypeptide chain of any one of claims 1 to 6 that is glycosylated.

11. A nucleic acid encoding the polypeptide chain of any one of claims 1 to 6.

12. A polypeptide chain encoded by a gene comprising:
(a) the DNA sequence described by Seq. ID No. 3;
(b) the DNA sequence described by Seq. ID No. 5;
(c) a DNA sequence hybridizable under stringent hybridization conditions with nucleotides 467-771 of Seq. ID No. 3 and encoding a polypeptide chain comprising an amino acid sequence which either: (a) shares the conserved six cysteine skeleton depicted in Fig. 2.1 to 2.3 from Leu (position 43) to His (position 139) of hOP1 and further comprises en additional cysteine residue in said six cysteine skeleton, or (b) shares the conserved seven cysteine skeleton depicted in Fig. 2.1 to 2.3 from Cys (position 38) to His (position 139) of hOP1 and further comprises an additional cysteine residue therein.
such that the dimeric osteogenic protein comprising said polypeptide chain has a conformation capable of inducing endochondral bone formation when implanted in a mammal in association with a matrix.

13. An isolated antibody, e.g. a polyclonal or monoclonal antibody, having binding specificity only for a polypeptide or protein as defined in any one of claims 1-10 and 12.

14. The antibody of claim 13 having binding specificity for a protein comprising an amino acid sequence described by:
(a) residues 303-399 of Seq. ID No. 5;
(b) residues 297-399 of Seq. ID No. 5;
(c) residues 264-399 of Seq. ID No. 5;
(d) residues 1-399 of Seq. ID No. 5;
(e) residues 18-257 of Seq. ID No. 5;
(f) residues 301-397 of Seq. ID No. 3;
(g) residues 296-397 of Seq. ID No. 3;
(h) residues 259-397 of Seq. ID No. 3;
(i) residues 1-397 of Seq. ID No. 3; or
(j) residues 18-259 of Seq. ID No. 3.

15. A DNA molecule encoding the protein of claim 12.

16. The DNA molecule of claim 15 wherein said molecule comprises:
(a) the DNA sequence described by Seq. ID No. 3;
(b) the DNA sequence described by Seq. ID No. 5; or
(c) the DNA sequence hybridizable with nuccleotides 467-771 of Seq. ID No. 3.

17. A protein comprising a pair of polypeptide chains and competent to induce cartilage or bone formation in a mammal, each of said polypeptide chains comprising the amino acid sequence defined by OPX (Seq. ID No. 7), wherein Xaa at residue 41 is Cys.

18. The protein of claim 7 or 12 in combination with a biocompatible, *in vivo* biodegradable carrier, said carrier acting as a slow release delivery system.

19. An implantable osteogenic device comprising the polypeptide chain of any one of claims 1-6, 8-10 and 12, or the protein of any one of claims 7, 17 and 18.

20. An osteogenic device for implantation in a mammal, the device comprising:
(a) a biocompatible, in vivo biodegradable matrix having pores of a dimension sufficient to permit the influx, differentiation and proliferation of migratory progenitor cells from the body of said mammal; and
(b) the polypeptide chain of any one of claims 1-6, 8-10 and 12, or the protein of any one of claims 7, 17 and 18.

21. The device of claim 20 wherein said matrix comprises allogenic or xenogenic bone.

22. The device of any one of claims 19-21 wherein said matrix comprises demineralized, delipidated, Type I insoluble bone collagen particles, depleted in noncollagenous protein, and treated to increase the intraparticle intrusion volume and surface area, for example with:
(a) a collagen fibril modifying agent; or
(b) a protease (e.g. trypsin); or
(c) a solvent (e.g. dichloromethane, trichloroacetic acid and acetonitrile); or
(d) an acid (e.g.trifluoroacetic acid or hydrogen fluoride); or
(e) a hot aqueous medium having a temperature within the range of 37 degrees centigrade to 75 degrees centigrade.

23. A process for producing the device of claim 22 comprising the step of treating collagen to increase the intraparticle intrusion volume and surface area.

24. The polypeptide chain of any one of claims 1-6, 8-10 and 12, or the protein of any one of claims 7, 17 and 18 in association with a matrix, wherein the matrix comprises:
(a) allogenic bone (for example, particulate, demineralized and guanidine extracted allogenic bone); or
(b) xenogenic bone (for example particulate, protein extracted, demineralized, xenogenic bone); or
(c) particulate, protein extracted, demineralized, xenogenic bone treated with a protease or a fibril modifying agent to increase the intraparticle intrusion volume and surface area, e.g. a solvent; or
(d) materials selected from collagen, homopolymers or copolymers of glycolic acid and lactic acid, hydroxyapatite and calcium phosphate (e.g. tricalcium phosphate) ; or
(e) a shape-retaining solid of loosely adhered particulate material, for example collagen; or
(f) a molded, porous solid; or
(g) masticated tissue (e.g. muscle).

25. An active heterodimeric osteogenic protein comprising the polypeptide chain of any one of claims 1-6, 8-10 and 12, or the protein of any one of claims 7, 17 and 18.

26. The heterodimeric protein of claim 25 wherein the heterodimers are joined by disulphide bonds or otherwise associated.

27. A process for producing the heterodimeric protein of claim 25 or 26 comprising the step of oxidising and refolding two or more polypeptide chains.

## Patentansprüche

1. Polypeptidkette, die eine Aminosäure-Sequenz enthält, die die Gruppen 303-399 der Sequenz ID Nr. 5 enthält.

2. Polypeptidkette nach Anspruch 1, die eine Aminosäure-Sequenz enthält, die die Gruppen
a) 298-399 der Sequenz ID Nr. 5 oder
b) 267-399 der Sequenz ID Nr. 5 oder
c) 264-399 der Sequenz ID Nr. 5 oder
d) 240-399 der Sequenz ID Nr. 5 oder
e) 1-399 der Sequenz ID Nr. 5
enthält.

3. Polypeptidkette, die eine Aminosäure-Sequenz enthält, die die Gruppen 301-397 der Sequenz ID Nr. 3 enthält.

4. Polypeptidkette nach Anspruch 3, die eine Aminosäure-Sequenz enthält, die die Gruppen
a) 296-397 der Sequenz ID Nr. 3 oder
b) 259-397 der Sequenz ID Nr. 3 oder
c) 1-397 der Sequenz ID Nr. 3
enthält.

5. Polypeptidkette, verwendbar als Untereinheit für ein dimeres osteogenes Protein, das ein Paar disulfidgebundene Polypeptidketten, wobei die Polypeptidkette eine Aminosäure-Sequenz enthält, die entweder
a) das erhaltene sechs Cystein-Gerüst von menschlichem OP1, dargestellt in Fig. 2.1 bis 2.3 von Leu (Position 43) bis His (Position 139), umschließt und weiterhin eine zusätzliche Cystein-Gruppe in diesem sechs Cystein-Gerüst enthält oder
b) das erhaltene sieben Cystein-Gerüst von menschlichem OP1, dargestellt in Fig. 2.1 bis 2.3 von Cys (Position 38) bis His (Position 139), umschließt und weiterhin eine zusätzliche Cystein-Gruppe darin enthält,
so daß das diese Polypeptidkette enthaltende, dimere osteogene Protein einen Aufbau hat, der eine endochondrale Knochenbildung zu induzieren in der Lage ist, wenn er in Verbindung mit einer Matrix einem Säuger implantiert wird.

6. Polypeptidkette nach Anspruch 5, wobei die beschriebene Aminosäure-Sequenz die Gruppen
a) 261-399 der Sequenz ID Nr. 5 oder
b) 301-397 der Sequenz ID Nr. 3 oder
c) 259-397 der Sequenz ID Nr. 3 oder
d) 298-299 der Sequenz ID Nr. 5
enthält.

7. Dimeres osteogenes Protein, das eine endochondrale Knochenbildung in einem Säuger zu induzieren in der Lage ist, wenn es diesem Säuger in Verbindung mit einer Matrix implantiert wird, und ein Paar disulfidgebundene Polypeptidketten, die eine dimere Spezies bilden, enthält, wobei jede dieser Polypeptidketten die Polypeptidkette nach Anspruch 5 ist.

8. Polypeptidkette nach einem der Ansprüche 1 bis 6, hergestellt durch die Produktion neuer Gene recombinanter DNS in einer Wirtszelle, z. B. einer prokaryontischen Wirtszelle oder einer Wirtszelle eines Säugers.

9. Polypeptidkette nach Anspruch 8, wobei die Wirtszelle aus Wirtszellen von E. coli, CHO, COS, BSC, Saccharomyces oder myeloma ausgewählt ist.

10. Polypeptidkette nach einem der Ansprüche 1 bis 6, die glycosyliert ist.

11. Nukleinsäure, die die Polypeptidkette nach einem der Ansprüche 1 bis 6 codiert.

12. Polypeptidkette, die mit einem Gen codiert ist, das
a) die DNS-Sequenz der Sequenz ID Nr. 3;
b) die DNS-Sequenz der Sequenz ID Nr. 5;
c) eine unter zwingend hybridisierenden Bedingungen hybridisierbare DNS-Sequenz mit den Nucleotiden 467-771 der Sequenz ID Nr. 3 enthält und eine Polypeptidkette codiert, die eine Aminosäure-Sequenz enthält, die entweder
a) das erhaltene sechs Cystein-Gerüst von menschlichem OP1, dargestellt in Fig. 2.1 bis 2.3 von Leu (Position 43) bis His (Position 139), umschließt und weiterhin eine zusätzliche Cystein-Gruppe in diesem sechs Cystein-Gerüst enthält oder
b) das erhaltene sieben Cystein-Gerüst von menschlichem OP1, dargestellt in Fig. 2.1 bis 2.3 von Cys (Position 38) bis His (Position 139), umfaßt und weiterhin eine zusätzliche Cystein-Gruppe darin enthält,
so daß das dimere osteogene Protein, welches diese Polypeptidkette enthält, einen Aufbau hat, der eine endochondrale Knochenbildung zu induzieren in der Lage ist, wenn er in Verbindung mit einer Matrix einem Säuger implantiert wird.

13. Isolierter Antikörper, z. B. polyklonaler oder monoklonaler Antikörper, der eine Bindungsspezifität nur für ein Polypeptid oder ein Protein nach einem der Ansprüche 1 bis 10 und 12 aufweist.

14. Antikörper nach Anspruch 13, der eine Bindungsspezifität für ein Protein mit einer Aminosäure-Sequenz aufweist, die die Gruppen
a) 303-399 der Sequenz ID Nr. 5;
b) 297-399 der Sequenz ID Nr. 5;
c) 264-399 der Sequenz ID Nr. 5;
d) 1-399 der Sequenz ID Nr. 5;
e) 18-257 der Sequenz ID Nr. 5;
f) 301-397 der Sequenz ID Nr. 3;
g) 296-397 der Sequenz ID Nr. 3;
h) 259-397 der Sequenz ID Nr. 3;
i) 1-397 der Sequenz ID Nr. 3 oder
j) 18-259 der Sequenz ID Nr. 3
enthält.

15. DNS-Molekül, das das Protein nach Anspruch 12 codiert.

16. DNS-Molekül nach Anspruch 15, das
a) die DNS-Sequenz der Sequenz ID Nr. 3;
b) die DNS-Sequenz der Sequenz ID Nr. 5 oder
c) die hybridisierbare DNS-Sequenz mit den Nukleotiden 467-771 der Sequenz ID Nr. 3
enthält.

17. Protein, das für die Knorpel- oder Knochenbildung bei einem Säuger maßgeblich ist und ein Paar Polypeptidketten enthält, die beide eine durch OPX (Sequenz ID Nr. 7) definierte Aminosäure-Sequenz enthalten, wobei Xaa in der Gruppe 41 Cys ist.

18. Protein nach Anspruch 7 oder 12 in Kombination mit einem biokompatiblen, in vivo biologisch abbaubaren Träger, der als langsam freisetzendes Versorgungssystem wirkt.

19. Implantierbares osteogenes Mittel, das die Polypeptidkette nach einem der Ansprüche 1 bis 6, 8 bis 10 und 12 oder das Protein nach einem der Ansprüche 7, 17 und 18 enthält.

20. Osteogenes Mittel für die Implantation in einen Säuger, das
a) eine biokompaktible, in vivo biologisch abbaubare Matrix mit einer ausreichenden Porengröße für die Zufuhr, die Differenzierung und das Wachstum von umgesiedelten Vorläufer-Zellen aus dem Körper dieses Säugers und
b) die Polypeptidkette nach einem der Ansprüche 1 bis 6, 8 bis 10 und 12, oder das Protein nach einem der Ansprüche 7, 17 und 18
enthält.

21. Mittel nach Anspruch 20, wobei die Matrix allogenes oder xenogenes Knochenmaterial enthält.

22. Mittel nach einem der Ansprüche 19 bis 21, wobei die Matrix demineralisierte, delipidisierte, Typ I unlösliche Knochen-Kollagenpartikeln enthält, deren nicht kollagene Proteine abgereichert sind, und die zur Vergrößerung des intrapartikulären Eindringvolumens und der Oberfläche z. B. mit:
a) einem Kollagenfibrillen modifizierenden Mittel oder
b) einer Protease (z. B. Trypsin) oder
c) einem Lösungsmittel (z. B. Dichlormethan, Trichloressigsäure und Acetonitril) oder
d) einer Säure (z. B. Trifluoressigsäure oder Fluorwasserstoff) oder
e) einem heißen wäßrigen Medium mit einer Temperatur im Bereich von 37°C bis 75°C
behandelt sind.

23. Verfahren zur Herstellung des Mittels nach Anspruch 22 mit dem Schritt der Kollagenbehandlung zur Vergrößerung des intrapartikulären Eindringvolumens und der Oberfläche.

24. Polypeptidkette nach einem der Ansprüche 1 bis 6, 8 bis 10 und 12, oder Protein nach einem der Ansprüche 7, 17 und 18 in Verbindung mit einer Matrix, die
a) allogenes Knochenmaterial (z. B. partikelförmiges, demineralisiertes und Guanidin extrahiertes allogenes Knochenmaterial) oder
b) xenogenes Knochenmaterial (z. B. partikelförmiges, demineralisiertes, Protein extrahiertes xenogenes Knochenmaterial) oder
c) partikelförmiges, demineralisiertes, Protein extrahiertes xenogenes Knochenmaterial, das mit einer Protease oder einem Fibrillen modifizierenden Mittel zur Vergrößerung des intrapartikulären Eindringvolumens und der Oberfläche, z. B. einem Lösungsmittel, behandelt ist oder
d) aus Kollagen ausgewähltes Material, Homo- oder - Copolymeren von Hydroxyessigsäure und Milchsäure, Hydroxyapatit und Calciumphosphat (z. B. Tricalciumphosphat) oder
e) ein formstabiles, festes oder lose zusammenhängendes, partikelförmiges Material, z. B. Kollagen oder
f) einen geformten, porösen Feststoff oder
g) zerkleinertes Gewebe (z. B. Muskelgewebe)
enthält.

25. Aktives heterodimeres osteogenes Protein, das die Polypeptidkette nach einem der Ansprüche 1 bis 6, 8 bis 10 und 12, oder das Protein nach einem der Ansprüche 7, 17 und 18 enthält.

26. Heterodimeres Protein nach Anspruch 25, wobei die Heterodimere über Disulfidbindungen oder anderweitig gebunden sind.

27. Verfahren zur Herstellung des heterodimeren Proteins nach Anspruch 25 oder 26 durch Oxidieren und Falten von zwei oder mehr Polypeptidketten.

## Revendications

1. Chaîne polypeptidique comprenant une séquence d'acides aminés décrite par les résidus 303-399 de Seq. ID No. 5.

2. Chaîne polypeptidique selon la revendication 1, comprenant une séquence d'acides aminés décrite par:
(a) les résidus 298-399 de Seq. ID No. 5; ou
(b) les résidus 267-399 de Seq. ID No. 5; ou
(c) les résidus 264-399 de Seq. ID No. 5; ou
(d) les résidus 240-399 de Seq. ID No. 5; ou
(e) les résidus 1-399 de Seq. ID No. 5.

3. Chaîne polypeptidique comprenant une séquence d'acides aminés décrite par les résidus 301-397 de Seq. ID No. 3.

4. Chaîne polypeptidique selon la revendication 3, comprenant une séquence d'acides aminés décrite par:
(a) les résidus 296-397 de Seq. ID No. 3; ou
(b) les résidus 259-397 de Seq. ID No. 3; ou
(c) les résidus 1-397 de Seq. ID No. 3.

5. Chaîne polypeptidique utile comme sous-unité d'une protéine ostéogène dimère comprenant une paire de chaînes polypeptidiques à liaisons disulfures, lesdites chaînes polypeptidiques comprenant une séquence d'acides aminés qui, soit
(a) partage le squelette conservé de six résidus cystéine décrit dans les figures 2.1 à 2.3 depuis le résidu Leu (position 43) jusqu'au résidu His (position 139) de hOP1, et comprend en outre un résidu cystéine supplémentaire dans ledit squelette de six résidus cystéine, soit
(b) partage le squelette conservé de sept résidus cystéine décrits dans les figures 2.1 à 2.3 depuis le résidu Cys (position 38) jusqu'au residu His (position 139) de hOP1, et comprend en outre un résidu cystéine supplémentaire dans ledit squelette,
de telle sorte que la protéine ostéogène dimère comprenant ladite chaîne polypeptidique possède une conformation capable d'induire une formation d'os cartilagineux lorsqu'elle est implantée dans un mammifère en association avec une matrice.

6. Chaîne polypeptidique selon la revendication 5, dans laquelle ladite séquence d'acides aminés comprend:
(a) les résidus 261-399 de Seq. ID No. 5; ou
(b) les résidus 301-397 de Seq. ID No. 3; ou
(c) les résidus 259-397 de Seq. ID No. 3; ou
(d) les résidus 298-399 de Seq. ID No. 5.

7. Protéine ostéogène dimère capable d'induire une formation d'os cartilagineux dans un mammifère lorsqu'elle est implantée dans ledit mammifère en association avec une matrice, ladite protéin comprenant une paire de chaînes polypeptidiques à liaisons disulfures Constituant une espèce dimère, dans laquelle chacune desdites chaînes polypeptidiques est la chaîne polypeptidique selon la revendication 5.

8. Chaîne polypeptidique selon l'une quelconque des revendications 1 à 6, obtenue par expression d'ADN recombinant dans une cellule hôte, par exemple une cellule hôte procaryote ou une cellule hôte mammalienne.

9. Chaîne polypeptidique selon la revendication 8, dans laquelle ladite cellule hôte est choisie parmi des cellules hôtes *E. coli,* CHO, COS, BSC, *Saccharomyces* ou de myélomes.

10. Chaîne polypeptidique selon l'une quelconque des revendications 1 à 6, à l'état glycosylé.

11. Acide nucléique encodant la chaîne polypeptidique selon l'une quelconque des revendications 1 à 6.

12. Chaîne polypeptidique encodée par un gène comprenant:
(a) la séquence d'ADN décrite par Seq. ID No. 3;
(b) la séquence d'ADN décrite par Seq. ID No. 5;
(c) une séquence d'ADN apte à s'hybrider dans des conditions d'hybridation sévères avec les nucléotides 467-771 de Seq. ID No. 3 et encodant une chaîne polypeptidique comprenant une séquence d'acides aminés qui, soit:
a) partage le squelette conservé de six résidus cystéine décrits dans les figures 2.1 à 2.3 depuis le résidu Leu (position 43) jusqu'au résidu His (position 139) de hOP1, et comprend en outre un résidu cystéine; supplémentaire dans ledit squelette de six résidus cystéine;
b) soit partage le squelette conservé de sept résidus cystéine décrits dans les figures 2.1 à 2.3 depuis le résidu Cys (position 38) jusqu'au résidu His (position 139) de hOP1, et comprend en outre un résidu cystéine supplémentaire dans ledit squelette,
de telle sorte que la protéine ostéogène dimère comprenant ladite chaîne polypeptidique possède une conformation capable d'induire une formation d'os cartilagineux lorsqu'elle est implantée dans un mammifère en association avec une matrice.

13. Anticorps isolé, par exemple anticorps polyclonal ou monoclonal possédant une spécificité de liaison uniquement pour un polypeptide ou pour une protéine tels que définis dans l'une quelconque des revendications 1-10 et 12.

14. Anticorps selon la revendication 13, possédant une spécificité de liaison pour une protéine comprenant une séquence d'acides aminés décrite par:
(a) les résidus 303-399 de Seq. ID No. 5;
(b) les résidus 297-399 de Seq. ID No. 5;
(c) les résidus 264-399 de Seq. ID No. 5;
(d) les résidus 1-399 de Seq. ID No. 5;
(e) les résidus 18-257 de Seq. ID No. 5;
(f) les résidus 301-397 de Seq. ID No. 3;
(g) les résidus 296-397 de Seq. ID No. 3;
(h) les résidus 259-397 de Seq. ID Nc. 3;
-(i) les résidus 1-397 de Seq. ID No. 3; ou
(j) les résidus 18-259 de Seq. ID No. 3.

15. Molécule d'ADN encodant la protéine selon la revendication 12.

16. Molécule d'ADN selon la revendication 15, dans laquelle ladite molécule comprend:
(a) la séquence d'ADN décrite par Seq. ID No. 3;
(b) la séquence d'ADN décrite par Seq. ID No. 5; ou
(c) la séquence d'ADN apte à s'hybrider avec les nucléotides 467-771 de Seq. ID No. 3.

17. Protéine comprenant une paire de chaînes polypeptidiques et compétente pour induire une formation d'os ou de cartilage dans un mammifère, chacune desdites chaînes polyeptidiques comprenant la séquence d'acides aminés définie par OPX (Seq. ID No. 7) dans laquelle Xaa au résidu 41 représente un résidu Cys.

18. Protéine selon la revendication 7 ou 12, en combinaison avec un support biocompatible biodégradable in vivo, ledit support faisant office de système à libération lente.

19. Dispositif ostéogène implantable comprenant la chaîne polypeptidique selon l'une quelconque des revendications 1 à 6, 8-10 et 12, ou la protéine selon l'une quelconque des revendications 7, 17 et 18.

20. Dispositif ostéogène destiné à une implantation dans un mammifère, le dispositif comprenant:
(a) une matrice biocompatible biodégradable in vivo possédant des pores de dimensions suffisantes pour permettre le flux entrant, la différenciation et la prolifération de cellules souches migratoires à partir du corps dudit mammifère; et
(b) la chaîne polypeptidique selon, l'une quelconque des revendications 1 à 6, 8 à 10 et 12, ou la protéine selon l'une quelconque des revendications 7, 17 et 18.

21. Dispositif selon la revendication 20, dans lequel ladite matrice comprend de l'os allogénique ou xénogène.

22. Dispositif selon l'une quelconque des revendications 19 à 21, dans lequel ladite matrice comprend des particules de collagène osseux insolubles de type I, déminéralisées, délipidées, appauvries en protéines non collagéniques et traitées pour augmenter le volume d'intrusion intraparticulaire et l'aire de surface, par exemple avec:
(a) un modificateur des fibrilles du collagène;
(b) une protéase (par exemple la trypsine); ou
(c) un solvant (par exemple le dichlorométhane, l'acide trichloracétique et l'acétonitrile) ; ou
(d) un acide (par exemple l'acide trifluoracétique ou l'acide fluorhydrique) ; ou
(e) un milieu aqueux chaud dont la température se situe dans le domaine de 37 degrés centigrades à 75 degrés centigrades.

23. Procédé pour préparer le dispositif selon la revendication 22, comprenant l'étape consistant à traiter du collagène pour augmenter le volume d'intrusion intraparticulaire et l'aire de surface.

24. Chaîne polypeptidique selon l'une quelconque des revendications 1 à 6, 8 à 10 et 12, ou protéine selon l'une quelconque des revendications 7, 17 et 18, en association avec une matrice, la matrice comprenant:
(a) de l'os allogénique (par exemple de l'os allogénique particulaire, déminéralisé et extrait dans de la guanidine); ou
(b) de l'os xénogène (par exemple de l'os xénogène, particulaire déminéralisé qui est extrait dans une protéine); ou
(c) de l'os xénogène particulaire, déminéralisé, extrait dans une protéine, traité avec une protéase ou avec un modificateur des fibrilles pour augmenter le volume d'intrusion intraparticulaire et l'aire de surface, par exemple un solvant; ou
(d) des matières choisies parmi le collagène, des homopolymères ou des copolymères de l'acide glycolique et de l'acide lactique, l'hydroxyapatite et le phosphate de calcium, par exemple le phosphate tricalcique); ou
(e) un produit solide indéformable d'une matière particulaire à adhérence lâche, par exemple le collagène; ou
(f) un produit solide poreux moulé; ou
(g) un tissu masticateur (par exemple un muscle).

25. Protéine active ostéogène hétérodimère comprenant la chaîne polypeptidique selon l'une quelconque des revendications 1 à 6, 8 à 10 et 12, ou la protéine selon l'une quelconque des revendications 7, 17 et 18.

26. Protéine hétérodimère selon la revendication 25, dans laquelle les hétérodimères sont joints par des liaisons disulfures ou associés d'une autre manière.

27. Procédé pour préparer la protéine hétérodimère selon la revendication 25 ou 26, comprenant l'étape consistant à soumettre à une oxydation et à un repliage deux chaînes polypeptidiques ou plus.
